# EUROPEAN PATENT APPLICATION

(11) **EP 4 834 938 A1**
(43) Date of publication of application: **04.11.2026**
(21) Application number: 24911535.3
(22) Date of filing: 27.12.2024
(51) Int. Cl.: C07K 16/28, C07K 16/30, C07K 16/46, G01N 33/574, A61K 39/00

(54) **MULTISPECIFIC ANTIBODY CONTAINING CCR8 ANTIGEN BINDING DOMAIN**

(30) Priority: 29.12.2023 CN 202311853250
(71) Applicant: Suzhou Zelgen Biopharmaceutical Co., Ltd., Jiangsu 215300 (CN); Shanghai Zelgen Pharma.Tech Co., Ltd., Shanghai 201203 (CN)
(72) Inventor: LV, Binhua, Shanghai 201203 (CN); DAI, Tongcheng, Shanghai 201203 (CN); LIN, Feng, Suzhou, Jiangsu 215300 (CN); SHENG, Zeqi, Suzhou, Jiangsu 215300 (CN); YAN, Shude, Suzhou, Jiangsu 215300 (CN); LIBAN, Tyler, Suzhou, Jiangsu 215300 (CN); ZHANG, Bin, Suzhou, Jiangsu 215300 (CN); SHENG, Zelin, Suzhou, Jiangsu 215300 (CN)
(74) Representative: dompatent
(86) International application number: PCT/CN2024/143352
(87) International publication number: WO 2025/140619

(57) **Abstract**

Provided are a multispecific antibody and the use thereof. Specifically, provided is a multispecific antibody, which contains a first targeting domain that targets a chemokine (C-C motif) receptor 8 (CCR8) molecule highly expressed on the surface of a tumor-infiltrating regulatory T cell (Treg), and a second targeting domain that binds to VEGF and/or a third targeting domain that binds to PD-Ll. Various functional domains of the CCR8-related multispecific antibody block a PD-1/PD-L1 immunosuppressive pathway, etc. by means of killing the tumor-infiltrating Treg, so that a T cell is activated, and the generation of a tumor vessel is inhibited. Therefore, the CCR8-related multispecific antibody can be used as an effective therapeutic agent for tumor treatment.

## Description

### TECHNICAL FIELD

The present invention relates to the field of cancer therapy, and more specifically, to a multispecific antibodies comprising an anti-CCR8 antibody or an immunoreactive fragment thereof for use in treating cancer.

### BACKGROUND

Cancer is generally defined as a group of diseases involving abnormal cell growth with the potential to invade or spread to other parts of the body. Conventional cancer therapies aim to remove cancerous tissue and prevent its metastasis. Such therapeutic options include surgery, chemotherapy, radiotherapy, hormone therapy, targeted therapy and palliative care. Treatment regimens are typically selected based on cancer type, location and grade, as well as the patient's physical condition and treatment preferences. However, these therapeutic approaches have inherent limitations: they may fail to work, especially when cancer has metastasized. Furthermore, chemotherapy and radiotherapy cause a spectrum of cytotoxicity-related side effects.

Promising areas in current cancer treatment encompass antibody-mediated targeted therapies and immunotherapies that harness the immune system to attack and eliminate tumor cells.

C-C Motif Chemokine Receptor 8 (CCR8) belongs to the G protein-coupled receptor (GPCR) superfamily and is a seven-transmembrane G protein-coupled protein. High CCR8 expression is negatively correlated with survival outcomes in multiple malignancies, including breast cancer, renal carcinoma, pancreatic cancer, bladder cancer, gastric cancer, cervical cancer and colon cancer. In cancer patients, CCR8 is highly expressed on tumor-resident regulatory T cells (Tregs) at tumor sites, with negligible expression in normal tissues and peripheral blood. Tumor-infiltrating Tregs constitute one of the major immunosuppressive cell populations within the tumor microenvironment. Anti-CCR8 antibodies deplete tumor-infiltrating Tregs via antibody-dependent cellular cytotoxicity (ADCC), effectively relieving T cell suppression and restoring the capacity of T cells to kill tumor cells. To date, no anti-CCR8 monoclonal antibodies or multispecific antibodies have been approved for marketing, yet multiple candidates have advanced into clinical research.

Antibodies of the IgG1 isotype exhibit potent ADCC activity, which is triggered by binding between the Fc region and Fc-γ receptors. The binding affinity is modulated by N-glycans within the CH2 domain. Research has demonstrated that reducing or eliminating fucose from the core Fc glycan structure enhances ADCC potency, and fucose synthesis is catalyzed by fucosyltransferase 8 (Fut8). Accordingly, knocking out the Fut8 gene in production cell lines such as CHO cells enables the expression of therapeutic antibodies with enhanced ADCC activity, which in turn improves Treg depletion and alleviates T cell suppression.

Vascular Endothelial Growth Factor (VEGF) is a member of the Platelet-Derived Growth Factor (PDGF) family. As a master mediator of tumor angiogenesis, VEGF drives continuous neovascularization within and around tumors. Tumor blood vessels formed under VEGF stimulation display structural and functional abnormalities that lead to poor tumor perfusion and hypoxia, which further upregulate VEGF production. Its critical role in tumor angiogenesis renders VEGF a well-established anti-tumor therapeutic target. Bevacizumab (brand name Avastin) is a monoclonal antibody developed by Genentech (a subsidiary of Roche) that specifically neutralizes VEGF to inhibit tumor angiogenesis. To date, bevacizumab has been approved for indications including colorectal cancer, non-small cell lung cancer, glioblastoma, renal cell carcinoma, cervical cancer, ovarian cancer, fallopian tube carcinoma, peritoneal carcinoma and the like.

PD-1/PD-L1 represents vital targets for immuno-oncology (IO) therapy. PD-L1 is overexpressed in most tumor types, and its binding to PD-1 on T cell surfaces transmits inhibitory signals to T cells. Blocking the PD-1/PD-L1 axis can robustly activate T cell-mediated tumor cell killing. Since the launch of Nivolumab, the first PD-1 antibody, in 2014, the development of PD-L1 antibodies has progressed rapidly. Among these, Atezolizumab developed by Roche obtained marketing approval in 2016, followed by Avelumab co-developed by Pfizer and Merck, which was approved in 2017.

Owing to the complexity of the tumor microenvironment, current monotherapy with monoclonal antibodies can hardly meet the growing clinical demands. There exists an urgent unmet clinical need in the field to develop multispecific antibodies that simultaneously target multiple anti-tumor therapeutic targets for superior therapeutic efficacy.

### SUMMARY OF THE INVENTION

The present invention constructs bispecific or trispecific antibodies to exert inhibitory or cytotoxic effects on tumor cells through distinct mechanisms simultaneously, thereby achieving superior therapeutic efficacy.

The present invention provides bispecific or trispecific antibodies comprising a CCR8 antigen-binding domain. Such bispecific or trispecific antibody molecules are capable of binding to CCR8 while concurrently blocking the VEGF pathway and/or the PD-L1 pathway. Also provided are methods for treating diseases such as cancer using the antibodies, antibody conjugates, pharmaceutical compositions and articles of manufacture of the present invention.

In the first aspect, it provides an anti-CCR8 antibody or an antigen-binding fragment thereof, wherein the antibody comprises the following three heavy chain variable region complementarity-determining regions (CDRs):
HCDR1 having an amino acid sequence set forth in SEQ ID NO: 1, 4, 7, 10, 15, 18, 20, 24, 28 or 30;
HCDR2 having an amino acid sequence set forth in SEQ ID NO: 2, 5, 8, 11, 13, 16, 21, 23, 25 or 31; and
HCDR3 having an amino acid sequence set forth in SEQ ID NO: 3, 6, 9, 12, 14, 17, 19, 22, 26, 27, 29 or 32;
and the following three light chain variable region CDRs:
   LCDR1 having an amino acid sequence set forth in SEQ ID NO: 33, 36, 39, 50 or 55;
   LCDR2 having an amino acid sequence set forth in SEQ ID NO: 34, 37, 40, 44, 48, 51, 53, 56 or 58; and
   LCDR3 having an amino acid sequence set forth in SEQ ID NO: 35, 38, 42, 45, 49, 52, 54 or 57.

In another preferred embodiment, the anti-CCR8 antibody or antigen-binding fragment thereof comprises three heavy chain variable region CDRs (HCDRs) and three light chain variable region CDRs (LCDRs) selected from the group consisting of:

| Antibody Name | HCDR1 Sequence No. | HCDR2 Sequence No. | HCDR3 Sequence No. | LCDR1 Sequence No. | LCDR2 Sequence No. | LCDR3 Sequence No. |
|---|---|---|---|---|---|---|
| C61 | 1 | 2 | 3 | 33 | 34 | 35 |
| C5 | 4 | 5 | 6 | 36 | 37 | 38 |
| C6 | 7 | 8 | 9 | 39 | 40 | 35 |
| C20 | 10 | 11 | 12 | 41 | 34 | 42 |
| C23 | 7 | 13 | 14 | 33 | 34 | 35 |
| C24 | 15 | 16 | 17 | 43 | 44 | 45 |
| C27 | 18 | 5 | 19 | 46 | 34 | 38 |
| C39 | 20 | 21 | 22 | 47 | 48 | 49 |
| C40 | 7 | 23 | 9 | 39 | 40 | 35 |
| C46 | 24 | 25 | 26 | 50 | 51 | 52 |
| C53 | 7 | 13 | 27 | 33 | 53 | 35 |
| C54 | 28 | 2 | 29 | 33 | 53 | 54 |
| C57 | 30 | 31 | 32 | 55 | 56 | 57 |
| C62 | 7 | 13 | 14 | 33 | 58 | 35. |

In another preferred embodiment, the anti-CCR8 antibody or antigen-binding fragment thereof comprises a heavy chain variable region having at least 80% sequence identity to an amino acid sequence set forth in any one of SEQ ID NO: 59 to 72, and/or a light chain variable region having at least 80% sequence identity to an amino acid sequence set forth in any one of SEQ ID NO: 73 to 86.

In another preferred embodiment, the anti-CCR8 antibody or antigen-binding fragment thereof comprises a heavy chain variable region set forth in SEQ ID NO: 59 and a light chain variable region set forth in SEQ ID NO: 73.

In another preferred embodiment, the anti-CCR8 antibody or antigen-binding fragment thereof comprises a heavy chain variable region set forth in SEQ ID NO: 60 and a light chain variable region set forth in SEQ ID NO: 74.

In another preferred embodiment, the anti-CCR8 antibody or antigen-binding fragment thereof comprises a heavy chain variable region set forth in SEQ ID NO: 61 and a light chain variable region set forth in SEQ ID NO: 75.

In another preferred embodiment, the anti-CCR8 antibody or antigen-binding fragment thereof comprises a heavy chain variable region set forth in SEQ ID NO: 62 and a light chain variable region set forth in SEQ ID NO: 76.

In another preferred embodiment, the anti-CCR8 antibody or antigen-binding fragment thereof comprises a heavy chain variable region set forth in SEQ ID NO: 63 and a light chain variable region set forth in SEQ ID NO: 77.

In another preferred embodiment, the anti-CCR8 antibody or antigen-binding fragment thereof comprises a heavy chain variable region set forth in SEQ ID NO: 64 and a light chain variable region set forth in SEQ ID NO: 78.

In another preferred embodiment, the anti-CCR8 antibody or antigen-binding fragment thereof comprises a heavy chain variable region set forth in SEQ ID NO: 65 and a light chain variable region set forth in SEQ ID NO: 79.

In another preferred embodiment, the anti-CCR8 antibody or antigen-binding fragment thereof comprises a heavy chain variable region set forth in SEQ ID NO: 66 and a light chain variable region set forth in SEQ ID NO: 80.

In another preferred embodiment, the anti-CCR8 antibody or antigen-binding fragment thereof comprises a heavy chain variable region set forth in SEQ ID NO: 67 and a light chain variable region set forth in SEQ ID NO: 81.

In another preferred embodiment, the anti-CCR8 antibody or antigen-binding fragment thereof comprises a heavy chain variable region set forth in SEQ ID NO: 68 and a light chain variable region set forth in SEQ ID NO: 82.

In another preferred embodiment, the anti-CCR8 antibody or antigen-binding fragment thereof comprises a heavy chain variable region set forth in SEQ ID NO: 69 and a light chain variable region set forth in SEQ ID NO: 83.

In another preferred embodiment, the anti-CCR8 antibody or antigen-binding fragment thereof comprises a heavy chain variable region set forth in SEQ ID NO: 70 and a light chain variable region set forth in SEQ ID NO: 84.

In another preferred embodiment, the anti-CCR8 antibody or antigen-binding fragment thereof comprises a heavy chain variable region set forth in SEQ ID NO: 71 and a light chain variable region set forth in SEQ ID NO: 85.

In another preferred embodiment, the anti-CCR8 antibody or antigen-binding fragment thereof comprises a heavy chain variable region set forth in SEQ ID NO: 72 and a light chain variable region set forth in SEQ ID NO: 86.

In another preferred embodiment, the antibody or antigen-binding fragment thereof is a human antibody, murine antibody, humanized antibody or chimeric antibody.

In another preferred embodiment, the antibody or antigen-binding fragment thereof is a human antibody.

In the second aspect, the present invention provides a multispecific antibody, wherein the multispecific antibody comprises the anti-CCR8 antibody or antigen-binding fragment thereof according to the first aspect of the present invention.

In another preferred embodiment, the multispecific antibody comprises:
a first targeting domain, wherein the first targeting domain comprises one or more CCR8 antigen-binding domains;
a second targeting domain, wherein the second targeting domain binds to VEGF or PD-L1;
optionally, a third targeting domain, wherein the third targeting domain binds to VEGF or PD-L1;
and the second targeting domain and the third targeting domain bind to different proteins respectively.

In another preferred embodiment, the targeting domain is in the form of a single-domain antibody (sdAb), variable fragment (Fv) heterodimer, single-chain Fv (scFv), Fab fragment, TriFab, or a combination thereof.

In another preferred embodiment, the CCR8 antigen-binding domain comprises the anti-CCR8 antibody or antigen-binding fragment thereof as described in the first aspect of the present invention.

In another preferred embodiment, the CCR8 antigen-binding domain comprises the following three heavy chain variable region CDRs:
HCDR1 having an amino acid sequence set forth in SEQ ID NO: 1;
HCDR2 having an amino acid sequence set forth in SEQ ID NO: 2; and
HCDR3 having an amino acid sequence set forth in SEQ ID NO: 3;
and the following three light chain variable region CDRs:
   LCDR1 having an amino acid sequence set forth in SEQ ID NO: 33;
   LCDR2 having an amino acid sequence set forth in SEQ ID NO: 34; and
   LCDR3 having an amino acid sequence set forth in SEQ ID NO: 35.

In another preferred embodiment, the CCR8 antigen-binding domain comprises the following three heavy chain variable region CDRs:
HCDR1 having an amino acid sequence set forth in SEQ ID NO: 18;
HCDR2 having an amino acid sequence set forth in SEQ ID NO: 5; and
HCDR3 having an amino acid sequence set forth in SEQ ID NO: 19;
and the following three light chain variable region CDRs:
   LCDR1 having an amino acid sequence set forth in SEQ ID NO: 46;
   LCDR2 having an amino acid sequence set forth in SEQ ID NO: 34; and
   LCDR3 having an amino acid sequence set forth in SEQ ID NO: 38.

In another preferred embodiment, the anti-CCR8 antibody or antigen-binding fragment thereof comprises a heavy chain variable region having at least 80% sequence identity to the amino acid sequence set forth in SEQ ID NO: 59, and/or a light chain variable region having at least 80% sequence identity to the amino acid sequence set forth in SEQ ID NO: 73.

In another preferred embodiment, the CCR8 antigen-binding domain comprises a heavy chain variable region set forth in SEQ ID NO: 59 and a light chain variable region set forth in SEQ ID NO: 73.

In another preferred embodiment, the anti-CCR8 antibody or antigen-binding fragment thereof comprises a heavy chain variable region having at least 80% sequence identity to the amino acid sequence set forth in SEQ ID NO: 65, and/or a light chain variable region having at least 80% sequence identity to the amino acid sequence set forth in SEQ ID NO: 79.

In another preferred embodiment, the CCR8 antigen-binding domain comprises a heavy chain variable region set forth in SEQ ID NO: 65 and a light chain variable region set forth in SEQ ID NO: 79.

In another preferred embodiment, the CCR8 antigen-binding domain is selected from the group consisting of scFv, Fab, and a combination thereof.

In another preferred embodiment, the CCR8 antigen-binding domain is an scFv.

In another preferred embodiment, the CCR8 antigen-binding domain is a Fab, which comprises a heavy chain variable region set forth in SEQ ID NO: 59 and a light chain variable region set forth in SEQ ID NO: 73, or a heavy chain variable region set forth in SEQ ID NO: 65 and a light chain variable region set forth in SEQ ID NO: 76; and
a heavy chain constant region CH1 set forth in SEQ ID NO: 111 or 119 and a light chain constant region CL set forth in SEQ ID NO: 121 or 122; or a heavy chain constant region CH1 set forth in SEQ ID NO: 112 and a light chain constant region CL set forth in SEQ ID NO: 123.

In another preferred embodiment, the multispecific antibody further comprises an Fc fragment.

In another preferred embodiment, the Fc fragment is derived from IgG1 or IgG4.

In another preferred embodiment, the Fc fragment is an IgG1-derived Fc fragment having an amino acid sequence set forth in SEQ ID NO: 113 or 114.

In another preferred embodiment, the Fc fragment comprises mutations for forming a knob-in-hole structure and/or mutations for enhancing ADCC.

In another preferred embodiment, the IgG1-derived Fc fragment comprises mutations selected from the group consisting of:
Y349C/K370E/K409D/K439E,
S354C/D356K/E357K/D399K;
S354C/T366W,
Y349C/T366S/L368A/Y407V.

In another preferred embodiment, the Fc fragment has an amino acid sequence set forth in any one of SEQ ID NO: 115 to 118.

In another preferred embodiment, the IgG4-derived Fc fragment comprises mutations selected from the group consisting of:
Y349C/K370E/R409D/K439E,
S354C/E356K/E357K/D399K; or
S354C/T366W,
Y349C/T366S/L368A/Y407V.

In another preferred embodiment, the Fc fragment is an IgG4-derived Fc fragment having an amino acid sequence set forth in SEQ ID NO: 120.

In another preferred embodiment, the multispecific antibody is a bispecific or trispecific antibody.

In another preferred embodiment, the multispecific antibody is a bispecific antibody.

In another preferred embodiment, the bispecific antibody comprises:
a first targeting domain, wherein the first targeting domain comprises one or more CCR8 antigen-binding domains; and a second targeting domain, wherein the second targeting domain is a VEGF antigen-binding domain.

In another preferred embodiment, the bispecific antibody comprises:
a first targeting domain, wherein the first targeting domain comprises one or more CCR8 antigen-binding domains; and a second targeting domain, wherein the second targeting domain is a PD-L1 antigen-binding domain.

In another preferred embodiment, the multispecific antibody is a trispecific antibody.

In another preferred embodiment, the trispecific antibody comprises:
a first targeting domain, wherein the first targeting domain comprises one or more CCR8 antigen-binding domains;
a second targeting domain, wherein the second targeting domain is a VEGF antigen-binding domain;
a third targeting domain, wherein the third targeting domain is a PD-L1 antigen-binding domain.

In another preferred embodiment, the VEGF antigen-binding domain comprises a heavy chain variable region having at least 80% sequence identity to the amino acid sequence set forth in SEQ ID NO: 87 or 88, and a light chain variable region having at least 80% sequence identity to the amino acid sequence set forth in SEQ ID NO: 93 or 94.

In another preferred embodiment, the VEGF antigen-binding domain comprises a heavy chain variable region having at least 80% sequence identity to the amino acid sequence set forth in SEQ ID NO: 89, and a light chain variable region having at least 80% sequence identity to the amino acid sequence set forth in SEQ ID NO: 95.

In another preferred embodiment, the VEGF antigen-binding domain comprises mutations capable of reducing antibody hydrophobicity.

In another preferred embodiment, the mutations capable of reducing antibody hydrophobicity are located in non-CDR3 regions of the anti-VEGF antigen-binding domain having the heavy chain variable region set forth in SEQ ID NO: 89 and the light chain variable region set forth in SEQ ID NO: 95.

In another preferred embodiment, the mutations capable of reducing antibody hydrophobicity occur at amino acid positions selected from the group consisting of position 28, 30, 31, 32, 33, 35 of the heavy chain variable region set forth in SEQ ID NO: 89, and a combination thereof.

In another preferred embodiment, the mutations capable of reducing antibody hydrophobicity occur at amino acid positions selected from the group consisting of position 24, 49, 50, 51, 52, 53, 56 of the light chain variable region set forth in SEQ ID NO: 95, and a combination thereof.

In another preferred embodiment, the mutations capable of reducing antibody hydrophobicity are located within residues 46 to 57 of the light chain variable region set forth in SEQ ID NO: 95.

In another preferred embodiment, the mutations capable of reducing antibody hydrophobicity substitute the foregoing amino acid residues with hydrophilic amino acids, such as aspartic acid (D), glutamic acid (E), lysine (K) or arginine (R).

In another preferred embodiment, the mutation capable of reducing antibody hydrophobicity is at serine (S) at position 30 of the heavy chain variable region set forth in SEQ ID NO: 89; preferably, serine (S) at position 30 is substituted with aspartic acid (D), glutamic acid (E), lysine (K) or arginine (R).

In another preferred embodiment, the mutations capable of reducing antibody hydrophobicity are at serine (S) at position 50 and/or serine (S) at position 52 of the light chain variable region set forth in SEQ ID NO: 95; preferably, serine (S) at position 50 is substituted with aspartic acid (D), glutamic acid (E), lysine (K) or arginine (R), and/or serine (S) at position 52 is substituted with aspartic acid (D), glutamic acid (E), lysine (K) or arginine (R).

In another preferred embodiment, the VEGF antigen-binding domain comprises a heavy chain variable region having at least 80% sequence identity to an amino acid sequence set forth in any one of SEQ ID NO: 90-92 and 149-150.

In another preferred embodiment, the VEGF antigen-binding domain comprises a light chain variable region having at least 80% sequence identity to an amino acid sequence set forth in any one of SEQ ID NO: 96-102.

In another preferred embodiment, the VEGF antigen-binding domain is selected from the group consisting of scFv, Fab, and combinations thereof.

In another preferred embodiment, the PD-L1 antigen-binding domain comprises a heavy chain variable region having at least 80% sequence identity to the amino acid sequence set forth in SEQ ID NO: 103 or 104, and a light chain variable region having at least 80% sequence identity to the amino acid sequence set forth in SEQ ID NO: 107 or 108.

In another preferred embodiment, the PD-L1 antigen-binding domain comprises a heavy chain variable region having at least 80% sequence identity to the amino acid sequence set forth in SEQ ID NO: 105 or 106, and a light chain variable region having at least 80% sequence identity to the amino acid sequence set forth in SEQ ID NO: 109 or 110.

In another preferred embodiment, the PD-L1 antigen-binding domain is selected from the group consisting of scFv, Fab, and combinations thereof.

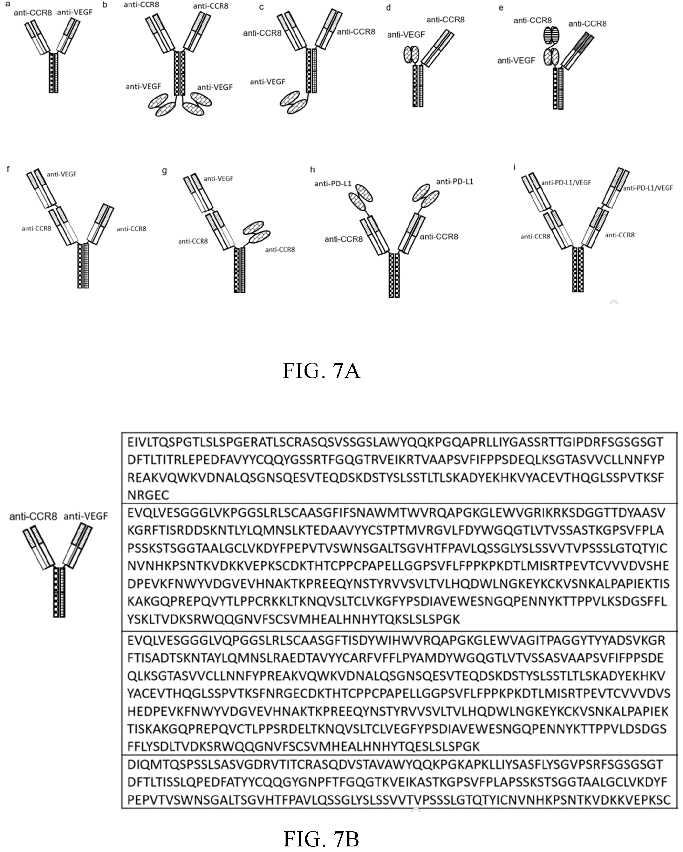

In another preferred embodiment, the multispecific antibody has a structure represented by Formula I (e.g., Figure 7A, a): 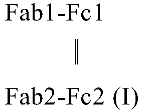
wherein each "-" independently represents a peptide bond or a linker peptide; and "∥" denotes an inter-polypeptide linkage;
Fab1 is a first targeting domain, and the Fab1 is an anti-CCR8 Fab;
Fab2 is a second targeting domain, and the Fab2 is an anti-VEGF Fab or an anti-PD-L1 Fab;
Fc1 and Fc2 each independently represent an Fc fragment.

In another preferred embodiment, the Fab2 is an anti-VEGF Fab.

In another preferred embodiment, the anti-CCR8 Fab comprises a heavy chain variable region set forth in SEQ ID NO: 59 and a light chain variable region set forth in SEQ ID NO: 73; or
the anti-CCR8 Fab comprises a heavy chain variable region set forth in SEQ ID NO: 65 and a light chain variable region set forth in SEQ ID NO: 79.

In another preferred embodiment, said anti-VEGF Fab comprises a heavy chain variable region set forth in SEQ ID NO: 87 or 88, and a light chain variable region set forth in SEQ ID NO: 93 or 94; or
the anti-VEGF Fab comprises a heavy chain variable region set forth in any one of SEQ ID NO: 89-92 and 149-150, and a light chain variable region set forth in any one of SEQ ID NO: 95-102.

In another preferred embodiment, said anti-PD-L1 Fab comprises a heavy chain variable region set forth in SEQ ID NO: 103 or 104, and a light chain variable region set forth in SEQ ID NO: 107 or 108; or
the anti-PD-L1 Fab comprises a heavy chain variable region set forth in SEQ ID NO: 105 or 106, and a light chain variable region set forth in SEQ ID NO: 109 or 110.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in any one of SEQ ID NO: 113-118.

In another preferred embodiment, the Fc1 has an amino acid sequence set forth in SEQ ID NO: 115, and the Fc2 has an amino acid sequence set forth in SEQ ID NO: 117.

In another preferred embodiment, the amino acid sequence of "HC1 (heavy chain)-Fc1" in "Fab1-Fc1" is set forth in SEQ ID NO: 125, and the amino acid sequence of "LC1 (light chain)" is set forth in SEQ ID NO: 124.

In another preferred embodiment, the amino acid sequence of "HC2 (heavy chain)-Fc2" in "Fab2-Fc2" is set forth in SEQ ID NO: 126, and the amino acid sequence of "LC2 (light chain)" is set forth in SEQ ID NO: 127.

In another preferred embodiment, the multispecific antibody has a structure represented by Formula II (e.g., Figure 7A, b): 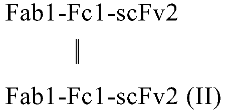 wherein each "-" independently represents a peptide bond or a linker peptide; and "∥" denotes an inter-polypeptide linkage;
Fab1 is a first targeting domain, and the Fab1 is an anti-CCR8 Fab;
scFv2 is a second targeting domain, and the scFv2 is an anti-VEGF scFv or an anti-PD-L1 scFv;
Fc1 represents an Fc fragment.

In another preferred embodiment, the "∥" represents a disulfide bond.

In another preferred embodiment, the scFv2 is an anti-VEGF scFv.

In another preferred embodiment, the anti-CCR8 Fab comprises a heavy chain variable region set forth in SEQ ID NO: 59 and a light chain variable region set forth in SEQ ID NO: 73; or
the anti-CCR8 Fab comprises a heavy chain variable region set forth in SEQ ID NO: 65 and a light chain variable region set forth in SEQ ID NO: 79.

In another preferred embodiment, the anti-VEGF scFv comprises a heavy chain variable region set forth in SEQ ID NO: 87 or 88, and a light chain variable region set forth in SEQ ID NO: 93 or 94; or
the anti-VEGF scFv comprises a heavy chain variable region set forth in any one of SEQ ID NO: 89-92 and 149-150, and a light chain variable region set forth in any one of SEQ ID NO: 95-102.

In another preferred embodiment, the anti-PD-L1 scFv comprises a heavy chain variable region set forth in SEQ ID NO: 103 or 104, and a light chain variable region set forth in SEQ ID NO: 107 or 108; or
the anti-PD-L1 scFv comprises a heavy chain variable region set forth in SEQ ID NO: 105 or 106, and a light chain variable region set forth in SEQ ID NO: 109 or 110.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in any one of SEQ ID NO: 113-118.

In another preferred embodiment, the Fc1 has an amino acid sequence set forth in SEQ ID NO: 113.

In another preferred embodiment, the amino acid sequence of "HC1 (heavy chain)-Fc1-scFv2" in "Fab1-Fc1-scFv2" is set forth in SEQ ID NO: 128, and the amino acid sequence of "LC1 (light chain)" is set forth in SEQ ID NO: 124.

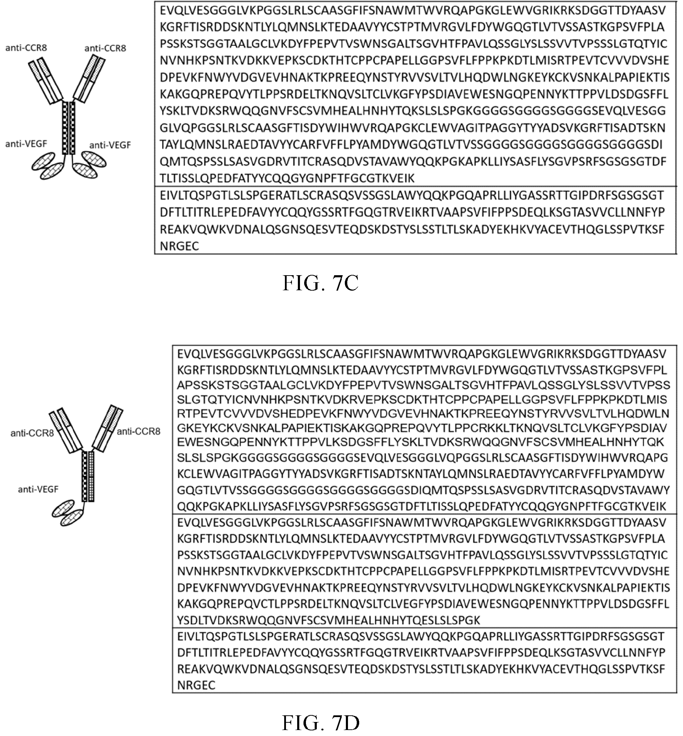

In another preferred embodiment, the multispecific antibody has a structure represented by Formula III (e.g., Figure 7A, c): 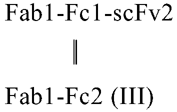
wherein each "-" independently represents a peptide bond or a linker peptide; and "∥" denotes an inter-polypeptide linkage;
Fab1 is a first targeting domain, and the Fab1 is an anti-CCR8 Fab;
scFv2 is a second targeting domain, and the scFv2 is an anti-VEGF scFv or an anti-PD-L1 scFv;
Fc1 and Fc2 each independently represent an Fc fragment.

In another preferred embodiment, the scFv2 is an anti-VEGF scFv.

In another preferred embodiment, the anti-CCR8 Fab comprises a heavy chain variable region set forth in SEQ ID NO: 59 and a light chain variable region set forth in SEQ ID NO: 73; or
the anti-CCR8 Fab comprises a heavy chain variable region set forth in SEQ ID NO: 65 and a light chain variable region set forth in SEQ ID NO: 79.

In another preferred embodiment, the anti-VEGF scFv comprises a heavy chain variable region set forth in SEQ ID NO: 87 or 88, and a light chain variable region set forth in SEQ ID NO: 93 or 94; or
the anti-VEGF scFv comprises a heavy chain variable region set forth in any one of SEQ ID NO: 89-92 and 149-150, and a light chain variable region set forth in any one of SEQ ID NO: 95-102.

In another preferred embodiment, the anti-PD-L1 scFv comprises a heavy chain variable region set forth in SEQ ID NO: 103 or 104, and a light chain variable region set forth in SEQ ID NO: 107 or 108; or
the anti-PD-L1 scFv comprises a heavy chain variable region set forth in SEQ ID NO: 105 or 106, and a light chain variable region set forth in SEQ ID NO: 109 or 110.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in any one of SEQ ID NO: 113-118.

In another preferred embodiment, the Fc1 has an amino acid sequence set forth in SEQ ID NO: 115, and the Fc2 has an amino acid sequence set forth in SEQ ID NO: 118.

In another preferred embodiment, the amino acid sequence of "HC1 (heavy chain)-Fc1-scFv2" in "Fab1-Fc1-scFv2" is set forth in SEQ ID NO: 128, and the amino acid sequence of "LC1 (light chain)" is set forth in SEQ ID NO: 124.

In another preferred embodiment, the amino acid sequence of "HC1 (heavy chain)-Fc2" in "Fab1-Fc2" is set forth in SEQ ID NO: 125, and the amino acid sequence of "LC1 (light chain)" is set forth in SEQ ID NO: 124.

In another preferred embodiment, the multispecific antibody has a structure represented by Formula IV (e.g., Figure 7A, d): 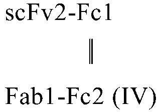
wherein each "-" independently represents a peptide bond or a linker peptide; and "∥" denotes an inter-polypeptide linkage;
Fab1 is a first targeting domain, and the Fab1 is an anti-CCR8 Fab;
scFv2 is a second targeting domain, and the scFv2 is an anti-VEGF scFv or an anti-PD-L1 scFv;
Fc1 and Fc2 each independently represent an Fc fragment.

In another preferred embodiment, the scFv2 is an anti-VEGF scFv.

In another preferred embodiment, the anti-CCR8 Fab comprises a heavy chain variable region set forth in SEQ ID NO: 59 and a light chain variable region set forth in SEQ ID NO: 73; or
the anti-CCR8 Fab comprises a heavy chain variable region set forth in SEQ ID NO: 65 and a light chain variable region set forth in SEQ ID NO: 79.

In another preferred embodiment, the anti-VEGF scFv comprises a heavy chain variable region set forth in SEQ ID NO: 87 or 88, and a light chain variable region set forth in SEQ ID NO: 93 or 94; or
the anti-VEGF scFv comprises a heavy chain variable region set forth in any one of SEQ ID NO: 89-92 and 149-150, and a light chain variable region set forth in any one of SEQ ID NO: 95-102.

In another preferred embodiment, the anti-PD-L1 scFv comprises a heavy chain variable region set forth in SEQ ID NO: 103 or 104, and a light chain variable region set forth in SEQ ID NO: 107 or 108; or the anti-PD-L1 scFv comprises a heavy chain variable region set forth in SEQ ID NO: 105 or 106 and a light chain variable region set forth in SEQ ID NO: 109 or 110.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in any one of SEQ ID NO: 113-118.

In another preferred embodiment, the Fc1 has an amino acid sequence set forth in SEQ ID NO: 115, and the Fc2 has an amino acid sequence set forth in SEQ ID NO: 118.

In another preferred embodiment, the amino acid sequence of "scFv2-Fc1" is set forth in SEQ ID NO: 129.

In another preferred embodiment, the amino acid sequence of "HC1 (heavy chain)-Fc2" in "Fab1-Fc2" is set forth in SEQ ID NO: 125, and the amino acid sequence of "LC1 (light chain)" is set forth in SEQ ID NO: 124.

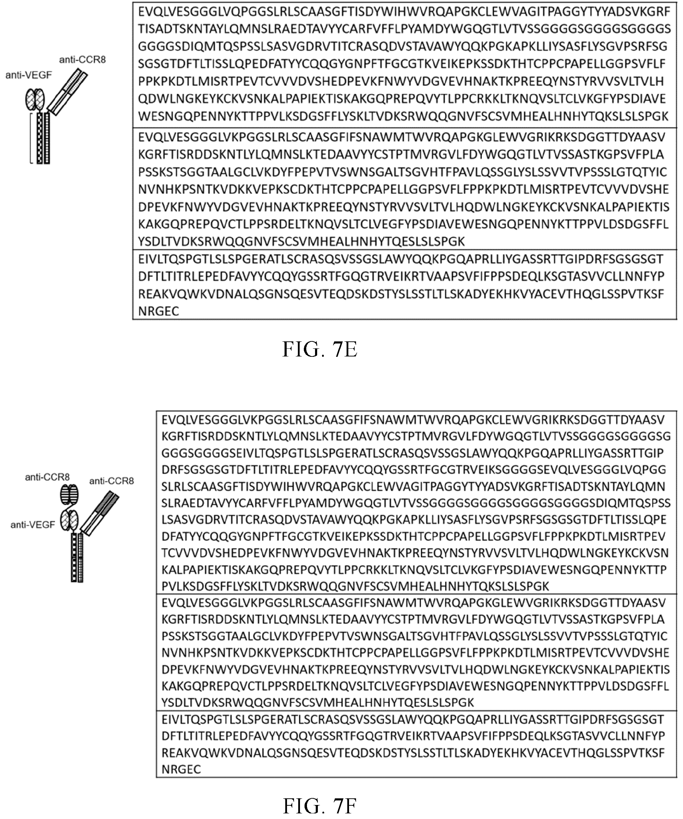

In another preferred embodiment, the multispecific antibody has a structure represented by Formula V (e.g., Figure 7A, e): 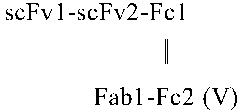
wherein each "-" independently represents a peptide bond or a linker peptide; and "∥" denotes an inter-polypeptide linkage;
scFv1 and Fab1 are first targeting domains, wherein the scFv1 is an anti-CCR8 scFv and the Fab1 is an anti-CCR8 Fab;
scFv2 is a second targeting domain, and the scFv2 is an anti-VEGF scFv or an anti-PD-L1 scFv;
Fc1 and Fc2 each independently represent an Fc fragment.

In another preferred embodiment, the scFv2 is an anti-VEGF scFv.

In another preferred embodiment, the anti-CCR8 Fab comprises a heavy chain variable region set forth in SEQ ID NO: 59 and a light chain variable region set forth in SEQ ID NO: 73; or
the anti-CCR8 Fab comprises a heavy chain variable region set forth in SEQ ID NO: 65 and a light chain variable region set forth in SEQ ID NO: 79.

In another preferred embodiment, the anti-CCR8 scFv comprises a heavy chain variable region set forth in SEQ ID NO: 59 and a light chain variable region set forth in SEQ ID NO: 73; or
the anti-CCR8 scFv comprises a heavy chain variable region set forth in SEQ ID NO: 65 and a light chain variable region set forth in SEQ ID NO: 79.

In another preferred embodiment, the anti-VEGF scFv comprises a heavy chain variable region set forth in SEQ ID NO: 87 or 88, and a light chain variable region set forth in SEQ ID NO: 93 or 94; or
the anti-VEGF scFv comprises a heavy chain variable region set forth in any one of SEQ ID NO: 89-92 and 149-150, and a light chain variable region set forth in any one of SEQ ID NO: 95-102.

In another preferred embodiment, the anti-PD-L1 scFv comprises a heavy chain variable region set forth in SEQ ID NO: 103 or 104, and a light chain variable region set forth in SEQ ID NO: 107 or 108; or
the anti-PD-L1 scFv comprises a heavy chain variable region set forth in SEQ ID NO: 105 or 106 and a light chain variable region set forth in SEQ ID NO: 109 or 110.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in any one of SEQ ID NO: 113-118.

In another preferred embodiment, the Fc1 has an amino acid sequence set forth in SEQ ID NO: 115, and the Fc2 has an amino acid sequence set forth in SEQ ID NO: 118.

In another preferred embodiment, the amino acid sequence of "scFv1-scFv2-Fc1" is set forth in SEQ ID NO: 130.

In another preferred embodiment, the amino acid sequence of "HC1 (heavy chain)-Fc2" in "Fab1-Fc2" is set forth in SEQ ID NO: 125, and the amino acid sequence of "LC1 (light chain)" is set forth in SEQ ID NO: 124.

In another preferred embodiment, the multispecific antibody has a structure represented by Formula VI (e.g., Figure 7A, f): 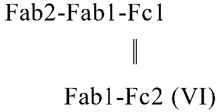
wherein each "-" independently represents a peptide bond or a linker peptide; and "∥" denotes an inter-polypeptide linkage;
Fab1 is a first targeting domain, and the Fab1 is an anti-CCR8 Fab;
Fab2 is a second targeting domain, and the Fab2 is an anti-VEGF Fab or an anti-PD-L1 Fab;
Fc1 and Fc2 each independently represent an Fc fragment.

In another preferred embodiment, the Fab2 is an anti-VEGF Fab.

In another preferred embodiment, the anti-CCR8 Fab comprises a heavy chain variable region set forth in SEQ ID NO: 59 and a light chain variable region set forth in SEQ ID NO: 73; or
the anti-CCR8 Fab comprises a heavy chain variable region set forth in SEQ ID NO: 65 and a light chain variable region set forth in SEQ ID NO: 79.

In another preferred embodiment, the anti-VEGF Fab comprises a heavy chain variable region set forth in SEQ ID NO: 87 or 88, and a light chain variable region set forth in SEQ ID NO: 93 or 94; or
the anti-VEGF Fab comprises a heavy chain variable region set forth in any one of SEQ ID NO: 89-92 and 149-150 and a light chain variable region set forth in any one of SEQ ID NO: 95-102.

In another preferred embodiment, the anti-PD-L1 Fab comprises a heavy chain variable region set forth in SEQ ID NO: 103 or 104, and a light chain variable region set forth in SEQ ID NO: 107 or 108; or
the anti-PD-L1 Fab comprises a heavy chain variable region set forth in SEQ ID NO: 105 or 106, and a light chain variable region set forth in SEQ ID NO: 109 or 110.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in any one of SEQ ID NO: 113-118.

In another preferred embodiment, the Fc1 has an amino acid sequence set forth in SEQ ID NO: 116, and the Fc2 has an amino acid sequence set forth in SEQ ID NO: 117.

In another preferred embodiment, the amino acid sequence of "HC2 (heavy chain) -HC1 (heavy chain)-Fc1" in "Fab2-Fab1-Fc1" is set forth in SEQ ID NO: 132, the amino acid sequence of "LC1 (light chain)" is set forth in SEQ ID NO: 131, and the amino acid sequence of "LC2 (light chain)" is set forth in SEQ ID NO: 134.

In another preferred embodiment, the amino acid sequence of "HC1 (heavy chain)-Fc2" in "Fab1-Fc2" is set forth in SEQ ID NO: 133, and the amino acid sequence of "LC1 (light chain)" is set forth in SEQ ID NO: 131.

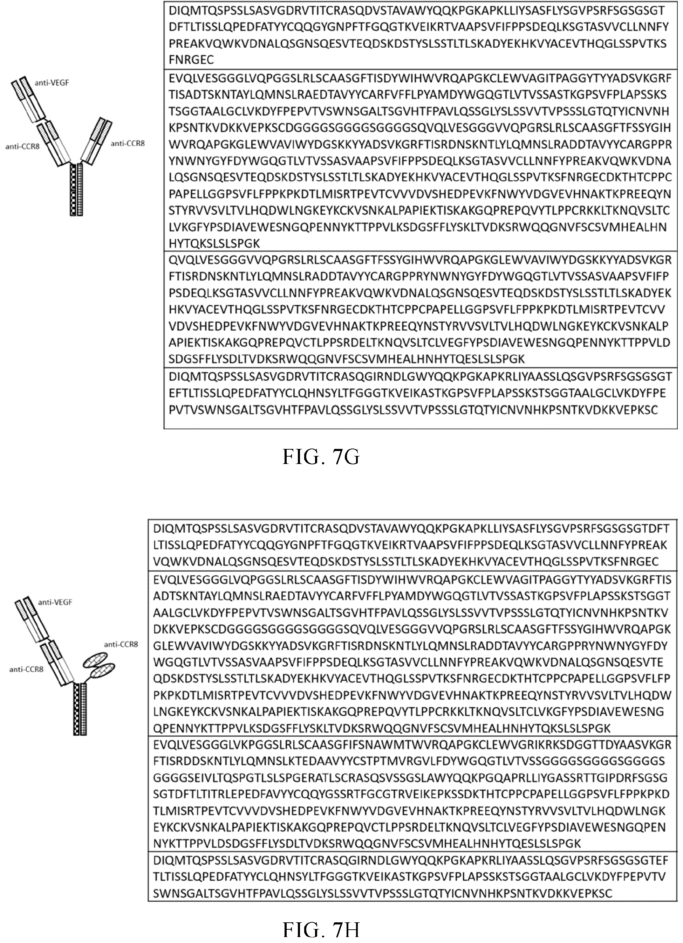

In another preferred embodiment, the multispecific antibody has a structure represented by Formula VII (e.g., Figure 7A, g): 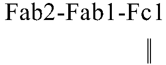 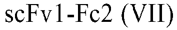
wherein each "-" independently represents a peptide bond or a linker peptide; and "∥" denotes an inter-polypeptide linkage;
Fab1 and scFv1 are first targeting domains, wherein the Fab1 is an anti-CCR8 Fab and the scFv1 is an anti-CCR8 scFv;
Fab2 is a second targeting domain, and said Fab2 is an anti-VEGF Fab or an anti-PD-L1 Fab;
Fc1 and Fc2 each independently represent an Fc fragment.

In another preferred embodiment, the Fab2 is an anti-VEGF Fab.

In another preferred embodiment, the anti-CCR8 Fab comprises a heavy chain variable region set forth in SEQ ID NO: 59 and a light chain variable region set forth in SEQ ID NO: 73; or
the anti-CCR8 Fab comprises a heavy chain variable region set forth in SEQ ID NO: 65 and a light chain variable region set forth in SEQ ID NO: 79.

In another preferred embodiment, the anti-CCR8 scFv comprises a heavy chain variable region set forth in SEQ ID NO: 59 and a light chain variable region set forth in SEQ ID NO: 73; or
the anti-CCR8 scFv comprises a heavy chain variable region set forth in SEQ ID NO: 65 and a light chain variable region set forth in SEQ ID NO: 79.

In another preferred embodiment, the anti-VEGF scFv comprises a heavy chain variable region set forth in SEQ ID NO: 87 or 88, and a light chain variable region set forth in SEQ ID NO: 93 or 94; or
the anti-VEGF scFv comprises a heavy chain variable region set forth in any one of SEQ ID NO: 89-92 and 149-150, and a light chain variable region set forth in any one of SEQ ID NO: 95-102.

In another preferred embodiment, said anti-PD-L1 scFv comprises a heavy chain variable region set forth in SEQ ID NO: 103 or 104, and a light chain variable region set forth in SEQ ID NO: 107 or 108; or
the anti-PD-L1 scFv comprises a heavy chain variable region set forth in SEQ ID NO: 105 or 106, and a light chain variable region set forth in SEQ ID NO: 109 or 110.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in any one of SEQ ID NO: 113-118.

In another preferred embodiment, the Fc1 has an amino acid sequence set forth in SEQ ID NO: 116, and the Fc2 has an amino acid sequence set forth in SEQ ID NO: 118.

In another preferred embodiment, the amino acid sequence of "HC2 (heavy chain) -HC1 (heavy chain)-Fc1" in "Fab2-Fab1-Fc1" is set forth in SEQ ID NO: 132, the amino acid sequence of "LC1 (light chain)" is set forth in SEQ ID NO: 131, and the amino acid sequence of "LC2 (light chain)" is set forth in SEQ ID NO: 134.

In another preferred embodiment, the amino acid sequence of "scFv1-Fc2" is set forth in SEQ ID NO: 135.

In another preferred embodiment, the multispecific antibody has a structure represented by Formula VIII (e.g., Figure 7A, h): 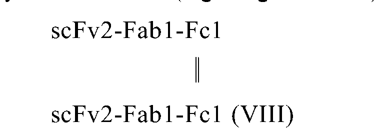
wherein each "-" independently represents a peptide bond or a linker peptide; and "∥" denotes an inter-polypeptide linkage;
Fab1 is a first targeting domain, and the Fab1 is an anti-CCR8 Fab;
scFv2 is a third targeting domain, and the scFv2 is an anti-PD-L1 scFv or an anti-VEGF scFv;
Fc1 represents an Fc fragment.

In another preferred embodiment, the "∥" represents a disulfide bond.

In another preferred embodiment, the anti-CCR8 Fab comprises a heavy chain variable region set forth in SEQ ID NO: 59 and a light chain variable region set forth in SEQ ID NO: 73; or
the anti-CCR8 Fab comprises a heavy chain variable region set forth in SEQ ID NO: 65 and a light chain variable region set forth in SEQ ID NO: 79.

In another preferred embodiment, the anti-PD-L1 scFv comprises a heavy chain variable region set forth in SEQ ID NO: 103 or 104, and a light chain variable region set forth in SEQ ID NO: 107 or 108; or
the anti-PD-L1 scFv comprises a heavy chain variable region set forth in SEQ ID NO: 105 or 106, and a light chain variable region set forth in SEQ ID NO: 109 or 110.

In another preferred embodiment, said anti-VEGF scFv comprises a heavy chain variable region set forth in SEQ ID NO: 87 or 88, and a light chain variable region set forth in SEQ ID NO: 93 or 94; or
the anti-VEGF scFv comprises a heavy chain variable region set forth in any one of SEQ ID NO: 89-92 and 149-150, and a light chain variable region set forth in any one of SEQ ID NO: 95-102.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in any one of SEQ ID NO: 113-118.

In another preferred embodiment, Fc1 has an amino acid sequence set forth in SEQ ID NO: 113.

In another preferred embodiment, the amino acid sequence of "scFv2-HC1 (heavy chain)-Fc1" in "scFv2-Fab1-Fc1" is set forth in SEQ ID NO: 136, and the amino acid sequence of "LC1 (light chain)" is set forth in SEQ ID NO: 124.

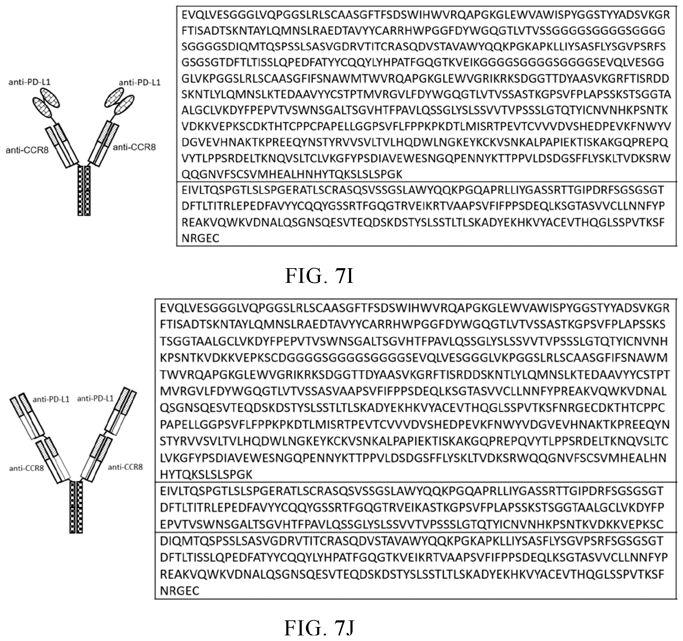

In another preferred embodiment, the multispecific antibody has a structure represented by Formula IX (e.g., Figure 7A, i): 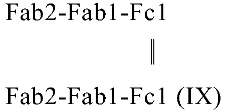
wherein each "-" independently represents a peptide bond or a linker peptide; and "∥" denotes an inter-polypeptide linkage;
Fab1 is a first targeting domain, and the Fab1 is an anti-CCR8 Fab;
Fab2 is a second targeting domain, and the Fab2 is an anti-PD-L1 Fab or an anti-VEGF Fab;
Fc1 represents an Fc fragment.

In another preferred embodiment, the "∥" represents a disulfide bond.

In another preferred embodiment, the anti-CCR8 Fab comprises a heavy chain variable region set forth in SEQ ID NO: 59 and a light chain variable region set forth in SEQ ID NO: 73; or
the anti-CCR8 Fab comprises a heavy chain variable region set forth in SEQ ID NO: 65 and a light chain variable region set forth in SEQ ID NO: 79.

In another preferred embodiment, the anti-PD-L1 Fab comprises a heavy chain variable region set forth in SEQ ID NO: 103 or 104, and a light chain variable region set forth in SEQ ID NO: 107 or 108; or
the anti-PD-L1 Fab comprises a heavy chain variable region set forth in SEQ ID NO: 105 or 106, and a light chain variable region set forth in SEQ ID NO: 109 or 110.

In another preferred embodiment, the anti-VEGF Fab comprises a heavy chain variable region set forth in SEQ ID NO: 87 or 88, and a light chain variable region set forth in SEQ ID NO: 93 or 94; or
the anti-VEGF Fab comprises a heavy chain variable region set forth in any one of SEQ ID NO: 89-92 and 149-150, and a light chain variable region set forth in any one of SEQ ID NO: 95-102.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in any one of SEQ ID NO: 113-118.

In another preferred embodiment, the Fc1 has an amino acid sequence set forth in SEQ ID NO: 114.

In another preferred embodiment, the amino acid sequence of "HC2 (heavy chain) -HC1 (heavy chain)-Fc1" in "Fab2-Fab1-Fc1" is set forth in SEQ ID NO: 138, the amino acid sequence of "LC1 (light chain)" is set forth in SEQ ID NO: 137, and the amino acid sequence of "LC2 (light chain)" is set forth in SEQ ID NO: 139.

In another preferred embodiment, the amino acid sequence of "HC2 (heavy chain) -HC1 (heavy chain)-Fc1" in "Fab2-Fab1-Fc1" is set forth in SEQ ID NO: 140, the amino acid sequence of "LC1 (light chain)" is set forth in SEQ ID NO: 137, and the amino acid sequence of "LC2 (light chain)" is set forth in SEQ ID NO: 134.

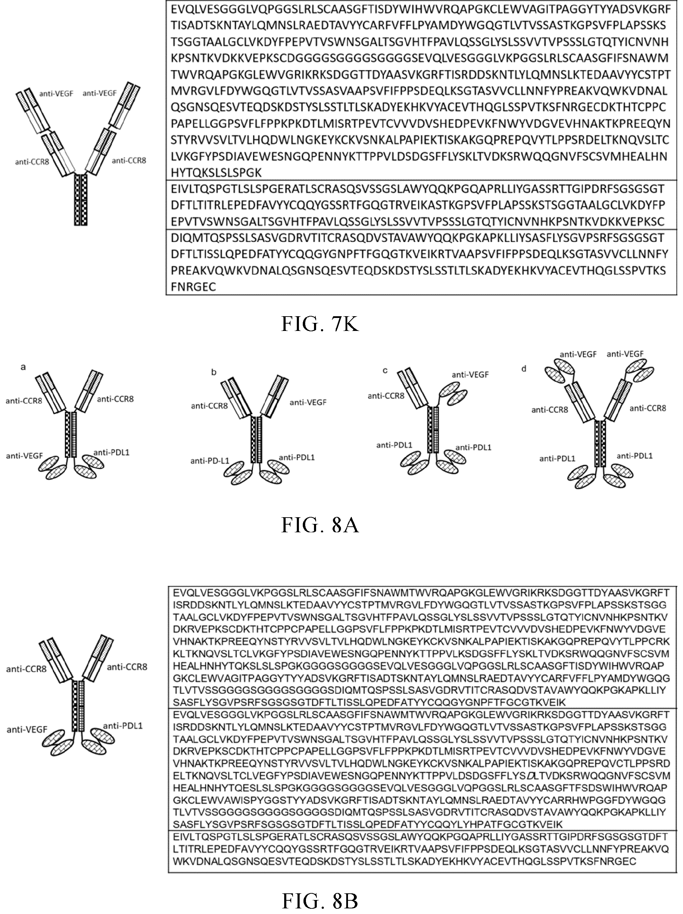

In another preferred embodiment, the multispecific antibody has a structure represented by Formula X (e.g., Figure 8A, a): 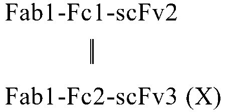
wherein each "-" independently represents a peptide bond or a linker peptide; and "∥" denotes an inter-polypeptide linkage;
Fab1 is a first targeting domain, and the Fab1 is an anti-CCR8 Fab;
scFv2 is a second targeting domain, and the scFv2 is an anti-VEGF scFv;
scFv3 is a third targeting domain, and the scFv3 is an anti-PD-L1 scFv;
Fc1 and Fc2 each independently represent an Fc fragment.

In another preferred embodiment, the anti-CCR8 Fab comprises a heavy chain variable region set forth in SEQ ID NO: 59 and a light chain variable region set forth in SEQ ID NO: 73; or
the anti-CCR8 Fab comprises a heavy chain variable region set forth in SEQ ID NO: 65 and a light chain variable region set forth in SEQ ID NO: 79.

In another preferred embodiment, the anti-VEGF scFv comprises a heavy chain variable region set forth in SEQ ID NO: 87 or 88, and a light chain variable region set forth in SEQ ID NO: 93 or 94; or
the anti-VEGF scFv comprises a heavy chain variable region set forth in any one of SEQ ID NO: 89-92 and 149-150, and a light chain variable region set forth in any one of SEQ ID NO: 95-102.

In another preferred embodiment, the anti-PD-L1 scFv comprises a heavy chain variable region set forth in SEQ ID NO: 103 or 104, and a light chain variable region set forth in SEQ ID NO: 107 or 108; or
the anti-PD-L1 scFv comprises a heavy chain variable region set forth in SEQ ID NO: 105 or 106, and a light chain variable region set forth in SEQ ID NO: 109 or 110.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in any one of SEQ ID NO: 113-118.

In another preferred embodiment, the Fc1 fragment has an amino acid sequence set forth in SEQ ID NO: 115, and the Fc2 fragment has an amino acid sequence set forth in SEQ ID NO: 118.

In another preferred embodiment, the amino acid sequence of "HC1 (heavy chain)-Fc1-scFv2" in "Fab1-Fc1-scFv2" is set forth in SEQ ID NO: 141, and the amino acid sequence of "LC1 (light chain)" is set forth in SEQ ID NO: 124.

In another preferred embodiment, the amino acid sequence of "HC1 (heavy chain)-Fc2-scFv3" in "Fab1-Fc2-scFv3" is set forth in SEQ ID NO: 142, and the amino acid sequence of "LC1 (light chain)" is set forth in SEQ ID NO: 124.

In another preferred embodiment, the multispecific antibody has a structure represented by Formula XI (Figure 8A, b): 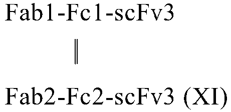
wherein each "-" independently represents a peptide bond or a linker peptide; and "∥" denotes an inter-polypeptide linkage;
Fab1 is a first targeting domain, and the Fab1 is an anti-CCR8 Fab;
Fab2 is a second targeting domain, and the Fab2 is an anti-VEGF Fab;
scFv3 is a third targeting domain, and the scFv3 is an anti-PD-L1 scFv;
Fc1 and Fc2 each independently represent an Fc fragment.

In another preferred embodiment, the anti-CCR8 Fab comprises a heavy chain variable region set forth in SEQ ID NO: 59 and a light chain variable region set forth in SEQ ID NO: 73; or
the anti-CCR8 Fab comprises a heavy chain variable region set forth in SEQ ID NO: 65 and a light chain variable region set forth in SEQ ID NO: 79.

In another preferred embodiment, the anti-VEGF Fab comprises a heavy chain variable region set forth in SEQ ID NO: 87 or 88, and a light chain variable region set forth in SEQ ID NO: 93 or 94; or
the anti-VEGF Fab comprises a heavy chain variable region set forth in any one of SEQ ID NO: 89-92 and 149-150, and a light chain variable region set forth in any one of SEQ ID NO: 95-102.

In another preferred embodiment, the anti-PD-L1 scFv comprises a heavy chain variable region set forth in SEQ ID NO: 103 or 104, and a light chain variable region set forth in SEQ ID NO: 107 or 108; or
the anti-PD-L1 scFv comprises a heavy chain variable region set forth in SEQ ID NO: 105 or 106, and a light chain variable region set forth in SEQ ID NO: 109 or 110.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in any one of SEQ ID NO: 113-118.

In another preferred embodiment, the Fc1 fragment has an amino acid sequence set forth in SEQ ID NO: 116, and the Fc2 fragment has an amino acid sequence set forth in SEQ ID NO: 118.

In another preferred embodiment, the amino acid sequence of "HC1 (heavy chain)-Fc1-scFv3" in "Fab1-Fc1-scFv3" is set forth in SEQ ID NO: 144, and the amino acid sequence of "LC1 (light chain)" is set forth in SEQ ID NO: 143.

In another preferred embodiment, the amino acid sequence of "HC1 (heavy chain)-Fc2-scFv3" in "Fab2-Fc2-scFv3" is set forth in SEQ ID NO: 145, and the amino acid sequence of "LC2 (light chain)" is set forth in SEQ ID NO: 134.

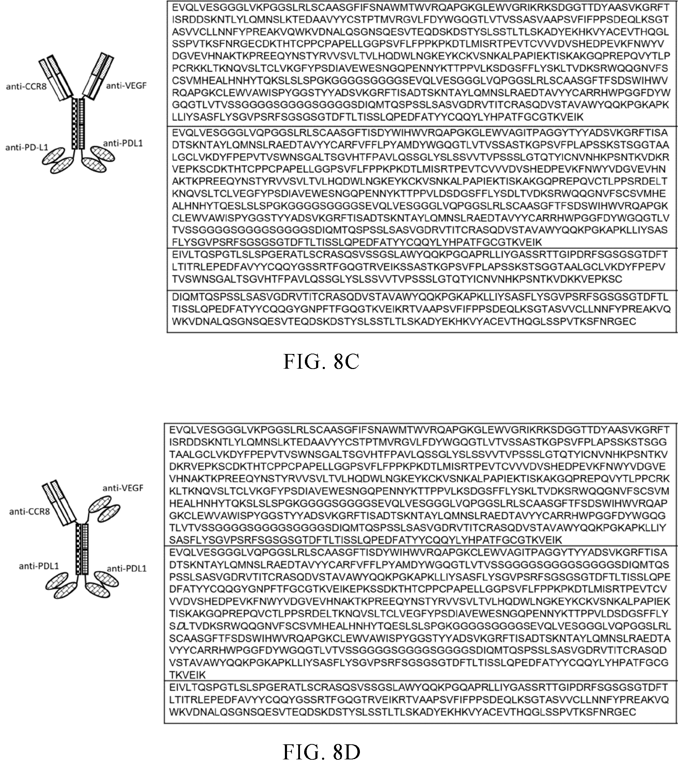

In another preferred embodiment, the multispecific antibody has a structure represented by Formula XII (e.g., Figure 8A, c): 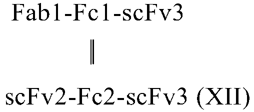
wherein each "-" independently represents a peptide bond or a linker peptide; and "∥" denotes an inter-polypeptide linkage;
Fab1 is a first targeting domain, and the Fab1 is an anti-CCR8 Fab;
scFv2 is a second targeting domain, and the scFv2 is an anti-VEGF scFv;
scFv3 is a third targeting domain, and the scFv3 is an anti-PD-L1 scFv;
Fc1 and Fc2 each independently represent an Fc fragment.

In another preferred embodiment, the anti-CCR8 Fab comprises a heavy chain variable region set forth in SEQ ID NO: 59 and a light chain variable region set forth in SEQ ID NO: 73; or
the anti-CCR8 Fab comprises a heavy chain variable region set forth in SEQ ID NO: 65 and a light chain variable region set forth in SEQ ID NO: 79.

In another preferred embodiment, the anti-VEGF scFv comprises a heavy chain variable region set forth in SEQ ID NO: 87 or 88, and a light chain variable region set forth in SEQ ID NO: 93 or 94; or
the anti-VEGF scFv comprises a heavy chain variable region set forth in any one of SEQ ID NO: 89-92 and 149-150, and a light chain variable region set forth in any one of SEQ ID NO: 95-102.

In another preferred embodiment, the anti-PD-L1 scFv comprises a heavy chain variable region set forth in SEQ ID NO: 103 or 104, and a light chain variable region set forth in SEQ ID NO: 107 or 108; or
the anti-PD-L1 scFv comprises a heavy chain variable region set forth in SEQ ID NO: 105 or 106, and a light chain variable region set forth in SEQ ID NO: 109 or 110.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in any one of SEQ ID NO: 113-118.

In another preferred embodiment, the Fc1 fragment has an amino acid sequence set forth in SEQ ID NO: 115, and the Fc2 fragment has an amino acid sequence set forth in SEQ ID NO: 118.

In another preferred embodiment, the amino acid sequence of "HC1 (heavy chain)-Fc1-scFv3" in "Fab1-Fc1-scFv3" is set forth in SEQ ID NO: 142, and the amino acid sequence of "LC1 (light chain)" is set forth in SEQ ID NO: 124.

In another preferred embodiment, the amino acid sequence of "scFv2-Fc2-scFv3" is set forth in SEQ ID NO: 146.

In another preferred embodiment, the multispecific antibody has a structure represented by Formula XIII (e.g., Figure 8A, d): 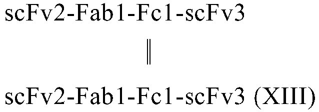
wherein each "-" independently represents a peptide bond or a linker peptide; and "∥" denotes an inter-polypeptide linkage;
Fab1 is a first targeting domain, and the Fab1 is an anti-CCR8 Fab;
scFv2 is a second targeting domain, and the scFv2 is an anti-VEGF scFv;
scFv3 is a third targeting domain, and the scFv3 is an anti-PD-L1 scFv;
Fc1 represents an Fc fragment.

In another preferred embodiment, the "∥" represents a disulfide bond.

In another preferred embodiment, the anti-CCR8 Fab comprises a heavy chain variable region set forth in SEQ ID NO: 59 and a light chain variable region set forth in SEQ ID NO: 73; or
the anti-CCR8 Fab comprises a heavy chain variable region set forth in SEQ ID NO: 65 and a light chain variable region set forth in SEQ ID NO: 79.

In another preferred embodiment, the anti-VEGF scFv comprises a heavy chain variable region set forth in SEQ ID NO: 87 or 88, and a light chain variable region set forth in SEQ ID NO: 93 or 94; or
the anti-VEGF scFv comprises a heavy chain variable region set forth in any one of SEQ ID NO: 89-92 and 149-150, and a light chain variable region set forth in any one of SEQ ID NO: 95-102.

In another preferred embodiment, the anti-PD-L1 scFv comprises a heavy chain variable region set forth in SEQ ID NO: 103 or 104, and a light chain variable region set forth in SEQ ID NO: 107 or 108; or
the anti-PD-L1 scFv comprises a heavy chain variable region set forth in SEQ ID NO: 105 or 106, and a light chain variable region set forth in SEQ ID NO: 109 or 110.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in any one of SEQ ID NO: 113-118.

In another preferred embodiment, the Fc1 fragment has an amino acid sequence set forth in SEQ ID NO: 113.

In another preferred embodiment, the amino acid sequence of "scFv2-HC1 (heavy chain)-Fc1-scFv3" in "scFv2-Fab1-Fc1-scFv3" is set forth in SEQ ID NO: 147, and the amino acid sequence of "LC1 (light chain)" is set forth in SEQ ID NO: 124.

In the third aspect, it provides a polynucleotide encoding the anti-CCR8 antibody or antigen-binding fragment thereof according to the first aspect of the present invention, or the multispecific antibody according to the second aspect of the present invention.

In the fourth aspect, it provides an expression vector comprising the polynucleotide according to the third aspect of the present invention.

In another preferred embodiment, the expression vector includes prokaryotic expression vectors and eukaryotic expression vectors.

In the fifth aspect, the present invention provides a host cell comprising the expression vector according to the fourth aspect of the present invention, or having the polynucleotide according to the third aspect of the present invention integrated into its genome.

In another preferred embodiment, the host cell is a prokaryotic cell or a eukaryotic cell.

In another preferred embodiment, the host cell is selected from the group consisting of *Escherichia coli*, yeast cells, HEK 293T cells, and CHO cells.

In the sixth aspect, it provides a use of the anti-CCR8 antibody or antigen-binding fragment thereof according to the first aspect of the present invention, or the multispecific antibody according to the second aspect of the present invention, for preparing a medicament for treating cancer/tumor.

In another preferred embodiment, the cancer/tumor is a CCR8-high-expressing cancer/tumor.

In another preferred embodiment, the cancer/tumor includes solid tumors or hematological malignancies.

In another preferred embodiment, the cancer/tumor is a solid tumor.

In another preferred embodiment, the cancer/tumor is selected from the group consisting of colorectal cancer, non-small cell lung cancer, glioblastoma, renal cell carcinoma, cervical cancer, ovarian cancer, fallopian tube carcinoma, peritoneal carcinoma, and combinations thereof.

In the seventh aspect, it provides an immunoconjugate, wherein the conjugate comprises:
(i) the anti-CCR8 antibody or antigen-binding fragment thereof according to the first aspect of the present invention, or the multispecific antibody according to the second aspect of the present invention; and
(ii) a coupling moiety selected from the group consisting of a detectable label, a drug, a toxin, a cytokine, a radionuclide, and an enzyme.

In another preferred embodiment, the coupling e moiety is selected from fluorescent or luminescent labels, radioactive labels, MRI (magnetic resonance imaging) or CT (computed tomography) contrast agents, enzymes capable of generating detectable products, radionuclides, biological toxins, cytokines (e.g., IL-2), antibodies, antibody Fc fragments, antibody scFv fragments, gold nanoparticles/nanorods, viral particles, liposomes, magnetic nanoparticles, prodrug-activating enzymes (e.g., DT-diaphorase (DTD) or biphenyl hydrolase-like protein (BPHL)), chemotherapeutic agents (e.g., cisplatin), nanoparticles of any form, and the like.

In an eighth aspect, it provides a pharmaceutical composition, comprising: (a) the anti-CCR8 antibody or antigen-binding fragment thereof according to the first aspect of the present invention, the multispecific antibody according to the second aspect of the present invention, or the immunoconjugate according to the seventh aspect of the present invention; and (b) a pharmaceutically acceptable carrier.

In another preferred embodiment, the pharmaceutical composition is in an injectable dosage form.

In the ninth aspect, it provides a method for treating cancer/tumor, comprising administering the multispecific antibody according to the first aspect of the present invention to a subject in need thereof.

In another preferred embodiment, the subject in need thereof is a human or non-human mammal.

In another preferred embodiment, the cancer/tumor is a CCR8-high-expressing cancer/tumor.

In another preferred embodiment, the cancer/tumor includes solid tumors or hematological malignancies.

In another preferred embodiment, the cancer/tumor is a solid tumor.

In another preferred embodiment, the cancer/tumor is selected from the group consisting of colorectal cancer, non-small cell lung cancer, glioblastoma, renal cell carcinoma, cervical cancer, ovarian cancer, fallopian tube carcinoma, peritoneal carcinoma, and combinations thereof.

In the tenth aspect, it provides a use of the anti-CCR8 antibody or antigen-binding fragment thereof according to the first aspect of the present invention, or the immunoconjugate according to the seventh aspect of the present invention, for preparing a detection reagent or a kit for detecting CCR8 molecules in a sample.

In another preferred embodiment, the sample includes an ex vivo sample, such as an ex vivo tissue or cell sample.

In another preferred embodiment, the detection reagent or kit serves as a diagnostic reagent for diagnosing CCR8-high-expressing cancer/tumor.

In the eleventh aspect, it provides an anti-VEGF antibody variant, wherein the anti-VEGF antibody variant comprises a heavy chain variable region having at least 80% sequence identity to the amino acid sequence set forth in SEQ ID NO: 89 and a light chain variable region having at least 80% sequence identity to the amino acid sequence set forth in SEQ ID NO: 95, and comprises mutations capable of reducing antibody hydrophobicity.

In another preferred embodiment, the mutations capable of reducing antibody hydrophobicity are located in non-CDR3 regions of the anti-VEGF antigen-binding domain having the heavy chain variable region set forth in SEQ ID NO: 89 and the light chain variable region set forth in SEQ ID NO: 95.

In another preferred embodiment, the mutations capable of reducing antibody hydrophobicity occur at amino acid positions selected from the group consisting of position 28, 30, 31, 32, 33, 35 of the heavy chain variable region set forth in SEQ ID NO: 89, or a combination thereof.

In another preferred embodiment, the mutations capable of reducing antibody hydrophobicity occur at amino acid positions selected from the group consisting of position 24, 49, 50, 51, 52, 53, 56 of the light chain variable region set forth in SEQ ID NO: 95, or a combination thereof.

In another preferred embodiment, the mutations capable of reducing antibody hydrophobicity are located within residues 46 to 57 of the light chain variable region set forth in SEQ ID NO: 95.

In another preferred embodiment, the mutations capable of reducing antibody hydrophobicity substitute one or more (e.g., two, three, four) of the foregoing amino acid residues with hydrophilic amino acids, such as aspartic acid (D), glutamic acid (E), lysine (K) or arginine (R).

In another preferred embodiment, the mutation capable of reducing antibody hydrophobicity is at serine (S) at position 30 of the heavy chain variable region set forth in SEQ ID NO: 89; preferably, serine (S) at position 30 is substituted with aspartic acid (D), glutamic acid (E), lysine (K) or arginine (R).

In another preferred embodiment, the mutations capable of reducing antibody hydrophobicity are at serine (S) at position 50 and/or serine (S) at position 52 of the light chain variable region set forth in SEQ ID NO: 95; preferably, serine (S) at position 50 is substituted with aspartic acid (D), glutamic acid (E), lysine (K) or arginine (R), and/or serine (S) at position 52 is substituted with aspartic acid (D), glutamic acid (E), lysine (K) or arginine (R).

In another preferred embodiment, the anti-VEGF antibody variant comprises a heavy chain variable region having at least 80% sequence identity to an amino acid sequence set forth in any one of SEQ ID NO: 90-92 and 149-150.

In another preferred embodiment, the anti-VEGF antibody variant comprises a light chain variable region having at least 80% sequence identity to an amino acid sequence set forth in any one of SEQ ID NO: 96-102.

In another preferred embodiment, the anti-VEGF antibody variant comprises a heavy chain variable region having the amino acid sequence set forth in SEQ ID NO: 91 and a light chain variable region having the amino acid sequence set forth in SEQ ID NO: 95.

In another preferred embodiment, the anti-VEGF antibody variant comprises a heavy chain variable region having the amino acid sequence set forth in SEQ ID NO: 89 and a light chain variable region having the amino acid sequence set forth in SEQ ID NO: 97.

In another preferred embodiment, the anti-VEGF antibody variant comprises a heavy chain variable region having the amino acid sequence set forth in SEQ ID NO: 89 and a light chain variable region having the amino acid sequence set forth in SEQ ID NO: 99.

In another preferred embodiment, the anti-VEGF antibody variant comprises a heavy chain variable region having the amino acid sequence set forth in SEQ ID NO: 89 and a light chain variable region having the amino acid sequence set forth in SEQ ID NO: 101.

In another preferred embodiment, the anti-VEGF antibody variant comprises a heavy chain variable region having the amino acid sequence set forth in SEQ ID NO: 91 and a light chain variable region having the amino acid sequence set forth in SEQ ID NO: 97.

In another preferred embodiment, the anti-VEGF antibody variant comprises a heavy chain variable region having the amino acid sequence set forth in SEQ ID NO: 91 and a light chain variable region having the amino acid sequence set forth in SEQ ID NO: 99.

In another preferred embodiment, the anti-VEGF antibody variant comprises a heavy chain variable region having the amino acid sequence set forth in SEQ ID NO: 91 and a light chain variable region having the amino acid sequence set forth in SEQ ID NO: 101.

In another preferred embodiment, compared with the parent antibody (i.e., the anti-VEGF antibody having the heavy chain variable region amino acid sequence of SEQ ID NO: 89 and the light chain variable region amino acid sequence of SEQ ID NO: 95), the anti-VEGF antibody variant exhibits significantly reduced antibody hydrophobicity and markedly diminished tendency of antibody molecular aggregation.

In another preferred embodiment, the term "significantly reduced antibody hydrophobicity" means that the hydrophobicity F1 of the anti-VEGF antibody variant satisfies F1/F0 < 1 relative to the hydrophobicity F0 of the parent antibody; preferably, F1/F0 ≤ 0.7, more preferably, F1/F0 ≤ 0.5.

In another preferred embodiment, the anti-VEGF antibody variant is used for constructing VEGF-targeted multispecific antibodies, such as the multispecific antibodies according to the second aspect of the present invention.

It should be understood that within the scope of the present invention, each technical features of the present invention described above and in the following (such as examples) may be combined with each other to form a new or preferred technical solution, which is not listed here due to space limitations.

### DESCRIPTION OF THE DRAWINGS

FIG. 1 shows FACS binding of CCR8 hybridoma monoclonal antibodies to human CCR8-expressing HEK293 cell line.
FIG. 2 shows the ADCC activity of recombinant anti-CCR8 monoclonal antibodies.
FIG. 3 shows the blocking effect of recombinant anti-CCR8 monoclonal antibodies on the binding between human CCR8 and human CCL1.
FIG. 4 is a schematic diagram illustrating surface hydrophobicity of the Fab structure of AS-1.
FIG. 5 shows aggregation propensity scores of all amino acid residues of the scFv of AS-1.
FIG. 6 shows peptide segments within the Fab sequence of AS-1 that exhibit aggregation propensity and high free energy.
FIG. 7A shows nine formats of bispecific antibodies composed of a CCR8 antigen-binding domain and a VEGF or PD-L1 antigen-binding domain (a-i);
   Wherein panel a shows an antibody structure formed by combination of an anti-VEGF Fab (CK-VH + CH1-VL) and an anti-CCR8 Fab (CH1-VH + CK-VL), in which IgG1 Fc forms a heterodimer via charge pair or knob-in-hole mutations; Panel b shows an antibody structure composed of an anti-CCR8 Fab and an anti-VEGF scFv (Fab-Fc-scFv × Fab-Fc-scFv); Panel c shows an antibody structure composed of an anti-CCR8 Fab and an anti-VEGF scFv (Fab-Fc-scFv × Fab-Fc); Panel d shows a molecular structure composed of an anti-VEGF scFv and an anti-CCR8 Fab (scFv-Fc × Fab-Fc); Panel e shows a molecular structure composed of an anti-CCR8 scFv, an anti-VEGF scFv and an anti-CCR8 Fab (scFv-scFv-Fc × Fab-Fc); Panel f shows an antibody structure formed by combination of an anti-VEGF Fab (CH1-VH + CK-VL), an anti-CCR8 Fab (CK-VH + CH1-VL) and another anti-CCR8 Fab (CK-VH + CH1-VL) (Fab-Fab-Fc × Fab-Fc); Panel g shows an antibody structure formed by combination of an anti-VEGF Fab (CH1-VH + CK-VL), an anti-CCR8 Fab (CK-VH + CH1-VL) and an anti-CCR8 scFv (Fab-Fab-Fc × scFv-Fc); Panel h shows an antibody structure formed by combination of an anti-PD-L1 scFv plus anti-CCR8 Fab and another anti-PD-L1 scFv plus anti-CCR8 Fab (scFv-Fab-Fc × scFv-Fab-Fc); Panel i shows an antibody structure formed by combination of an anti-VEGF or anti-PD-L1 Fab (CH1-VH + CK-VL) plus anti-CCR8 Fab (CK-VH + CH1-VL) and another anti-VEGF or anti-PD-L1 Fab (CH1-VH + CK-VL) plus anti-CCR8 Fab (CK-VH + CH1-VL) (Fab-Fab-Fc × Fab-Fab-Fc); wherein the IgG1 Fc regions form a heterodimer via charge pair or knob-in-hole mutations.
FIG. 7B shows an exemplary bispecific antibody structure corresponding to panel a in FIG. 7A, designated 8As-1.
FIG. 7C shows an exemplary bispecific antibody structure corresponding to panel b in FIG. 7A, designated 8As-2.
FIG. 7D shows an exemplary bispecific antibody structure corresponding to panel c in FIG. 7A, designated 8As-3.
FIG. 7E shows an exemplary bispecific antibody structure corresponding to panel d in FIG. 7A, designated 8As-4.
FIG. 7F shows an exemplary bispecific antibody structure corresponding to panel e in FIG. 7A, designated 8As-5.
FIG. 7G shows an exemplary bispecific antibody structure corresponding to panel f in FIG. 7A, designated 8As-6.
FIG. 7H shows an exemplary bispecific antibody structure corresponding to panel g in FIG. 7A, designated 8As-7.
FIG. 7I shows an exemplary bispecific antibody structure corresponding to panel h in FIG. 7A, designated Pl8-8.
FIG. 7J shows an exemplary bispecific antibody structure corresponding to panel i in FIG. 7A, designated Pl8-9.
FIG. 7K shows another exemplary bispecific antibody structure corresponding to panel i in FIG. 7A, designated 8As-9.
FIG. 8A shows three formats of trispecific antibodies composed of a CCR8 antigen-binding domain, a VEGF antigen-binding domain and a PD-L1 antigen-binding domain;
wherein panel a shows a molecular structure composed of an anti-CCR8 antibody, an anti-VEGF antibody and an anti-PD-L1 antibody (IgG-scFv × IgG-scFv); Panel b shows a molecular structure composed of an anti-CCR8 antibody, an anti-VEGF antibody and an anti-PD-L1 antibody (Fab-Fc-scFv × Fab-Fc-scFv); Panel c shows a molecular structure composed of an anti-CCR8 antibody, an anti-VEGF antibody and an anti-PD-L1 antibody (IgG-scFv × scFv-Fc-scFv); Panel d shows a molecular structure composed of an anti-CCR8 antibody, an anti-VEGF antibody and an anti-PD-L1 antibody (scFv-IgG-scFv × scFv-IgG-scFv).
FIG. 8B shows an exemplary trispecific antibody structure corresponding to panel a in FIG. 8A, designated 8AsPl-1.
FIG. 8C shows an exemplary trispecific antibody structure corresponding to panel b in FIG. 8A, designated 8AsPl-2.
FIG. 8D shows an exemplary trispecific antibody structure corresponding to panel c in FIG. 8A, designated 8AsPl-3.

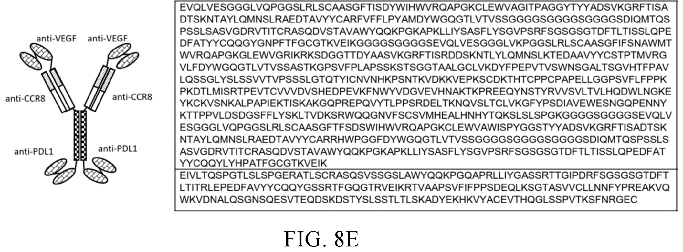

FIG. 8E shows an exemplary trispecific antibody structure corresponding to panel d in FIG. 8A, designated 8AsPl-4.

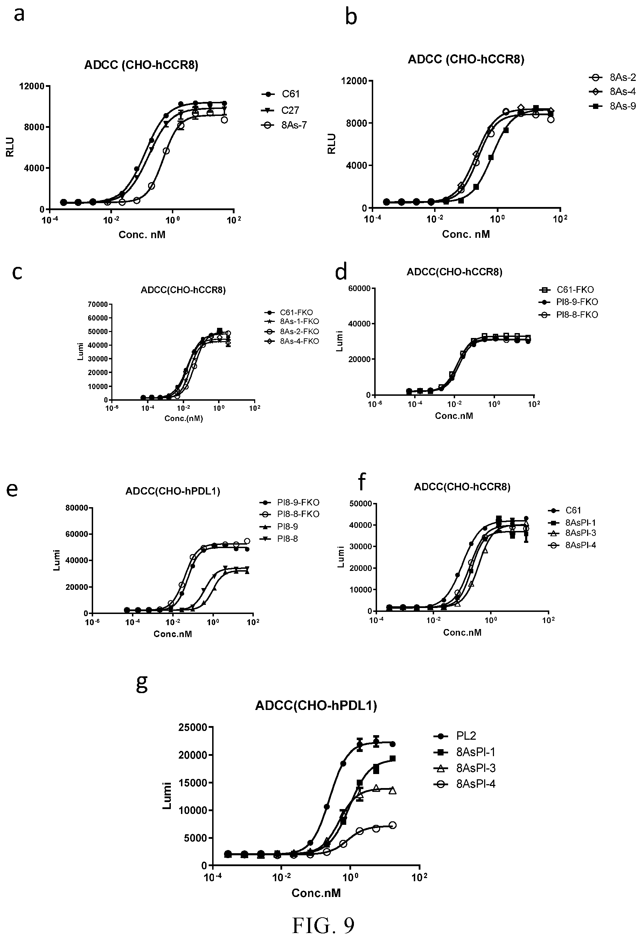

FIG. 9 shows the ADCC activity of the monoclonal antibodies and multispecific antibodies of the present invention.

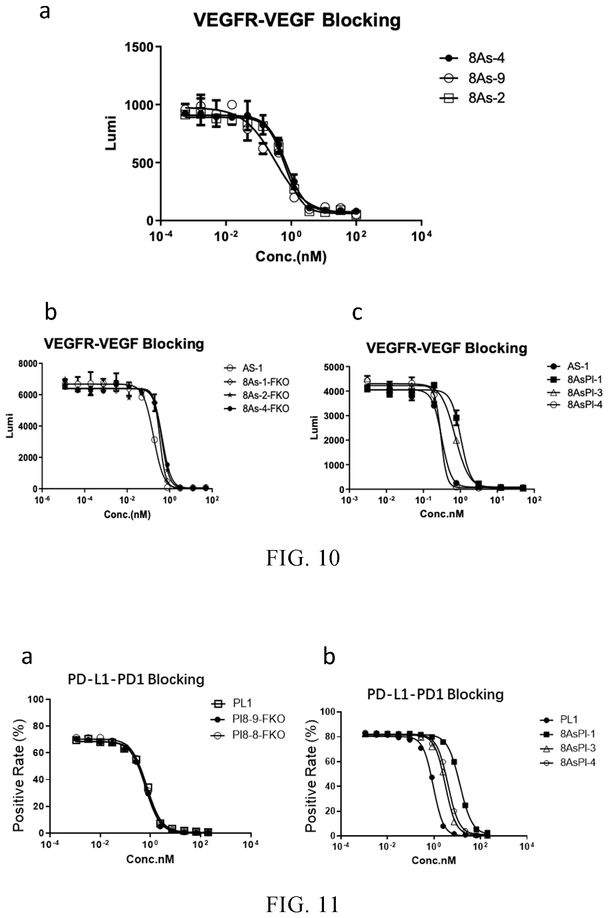

FIG. 10 shows the VEGF blocking activity of the multispecific antibodies of the present invention.
FIG. 11 shows the PD-L1 blocking activity of the multispecific antibodies of the present invention.

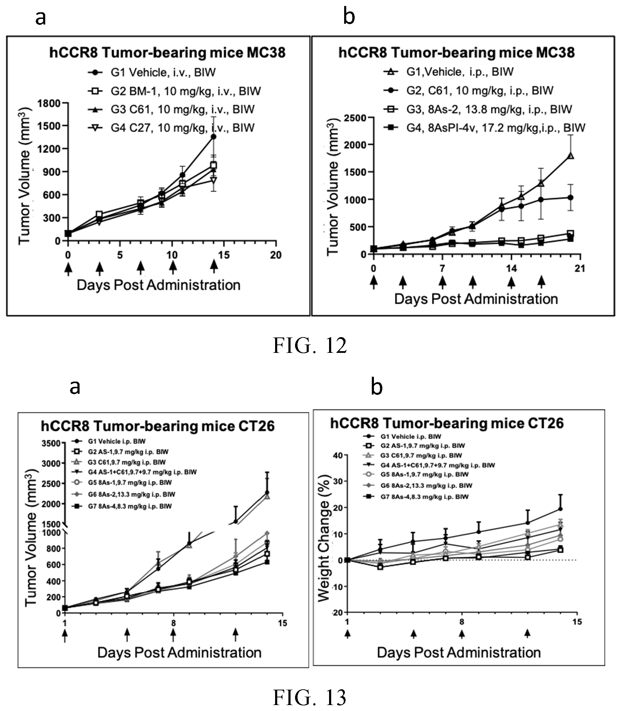

FIG. 12 shows the *in vivo* efficacy of the anti-CCR8 monoclonal antibody of the present invention in mouse tumor models.
FIG. 13 shows the *in vivo* efficacy of the anti-CCR8 bispecific antibody of the present invention in mouse tumor models.

### DETAILED DESCRIPTION

Through extensive and in-depth research, the present inventors have unexpectedly developed a class of multispecific antibodies comprising a CCR8 antigen-binding domain for the first time. The multispecific antibody contains a first targeting domain that targets chemokine (C-C motif) receptor 8 (CCR8) highly expressed on the surface of tumor-infiltrating regulatory T cells, wherein the first targeting domain is an anti-CCR8 antibody or an antigen-binding fragment thereof, and further comprises a second targeting domain and/or a third targeting domain that bind(s) to VEGF and/or PD-L1. The multispecific antibody of the present invention is capable of simultaneously binding to Treg cells, tumor cells and free VEGF molecules, and can serve as an effective therapeutic agent for tumor treatment.

On this basis, the present invention has been completed.

As used herein, the term "chemokine (C-C motif) receptor 8" or "CCR8" refers to the protein encoded by the CCR8 gene in humans. CCR8 is highly expressed on numerous tumor-infiltrating Treg cells, while exhibiting low or no expression on Treg cells from the thymus, spleen and peripheral blood.

VEGF (vascular endothelial growth factor) is a member of the platelet-derived growth factor (PDGF) family. VEGF acts as a key mediator of tumor angiogenesis. It mediates continuous neovascularization within and around tumors. Tumor blood vessels formed under the action of VEGF exhibit structural and functional abnormalities, which lead to poor tumor perfusion and hypoxia, thereby driving further production of VEGF. The critical role of VEGF in tumor angiogenesis renders it a well-established anti-tumor therapeutic target.

PD-1/PD-L1 represents an important target for immuno-oncology (IO) therapy. PD-L1 is overexpressed in most tumors. Binding of PD-L1 to PD-1 on the surface of T cells transmits inhibitory signals to T cells. Accordingly, blockade of the PD-1/PD-L1 axis can effectively activate T cell-mediated killing of tumor cells.

The term "Fc fragment" or "Fc" refers to the portion of an antibody that lacks antigen-binding activity and was initially observed to crystallize readily, hence named the Fc fragment (fragment crystallizable). This fragment corresponds to paired CH2 and CH3 domains and constitutes the region of an antibody molecule that interacts with effector molecules and cells. The Fc fragments described herein may be derived from IgG1, IgG2 and IgG4 antibodies. A particular IgG subclass may be preferred for specific applications. For example, IgG1 is more effective than IgG2 and IgG4 in mediating ADCC and CDC. Accordingly, IgG2 Fc may be preferred when effector functions are undesirable. Nevertheless, molecules comprising an IgG2 Fc are generally more difficult to produce and may be less stable than molecules containing an IgG1 Fc. Furthermore, effector functions of antibodies can be enhanced or attenuated by introducing one or more mutations into the Fc (see, e.g., Strohl, Curr. Opin. Biotechnol., 20:685-691, 2009).

As used herein, the term "antibody" or "immunoglobulin" is a heterotetrameric glycoprotein of approximately 150,000 daltons with identical structural characteristics, composed of two identical light chains (L) and two identical heavy chains (H). Each light chain is linked to a heavy chain via one covalent disulfide bond, whereas the number of disulfide bonds between heavy chains varies among different immunoglobulin isotypes. Each heavy and light chain also possesses regularly spaced intrachain disulfide bonds. Each heavy chain has a variable region (VH) at one terminus followed by multiple constant regions. Each light chain has a variable region (VL) at one terminus and a constant region at the other terminus; the light chain constant region aligns with the first constant region of the heavy chain, and the light chain variable region aligns with the heavy chain variable region. Specific amino acid residues form an interface between the variable regions of the light chain and the heavy chain.

As used herein, the term "variable" denotes that certain portions of the variable regions of an antibody differ in sequence, which confer binding capacity and specificity of each distinct antibody for its particular antigen. However, variability is not uniformly distributed across the entire antibody variable region. It is concentrated within three segments termed complementarity-determining regions (CDRs) or hypervariable regions in the light chain and heavy chain variable regions. The relatively conserved portions within the variable regions are referred to as framework regions (FRs). The variable regions of native heavy and light chains each contain four FRs, which adopt largely β-sheet conformations and are connected by three CDRs that form connecting loops, and in some instances may form part of the β-sheet structure. The CDRs within each chain are brought into close proximity via the FRs and, together with the CDRs from the other chain, constitute the antigen-binding site of the antibody (see Kabat et al., NIH Publ. No. 91-3242, Volume I, pp. 647-669 (1991)). Constant regions do not directly participate in antigen binding by the antibody, yet they exhibit diverse effector functions, such as mediating antibody-dependent cellular cytotoxicity.

The antibodies of the present application include, but are not limited to, polyclonal, monoclonal, monospecific, multispecific, bispecific, human, humanized, primatized, chimeric and single-chain antibodies. The antibodies disclosed herein may be derived from any animal source, including avian and mammalian species. Preferably, the antibodies are human, murine, donkey, rabbit, goat, guinea pig, camel, llama, horse or chicken antibodies.

The term "antibody fragment" or "antigen-binding fragment" refers to a portion of an antibody, such as F(ab')₂, F(ab)₂, Fab', Fab, Fv, single-chain Fvs (scFv), single-chain antibodies, disulfide-linked Fvs (sdFv), fragments comprising a VL or VH domain, fragments produced by Fab expression libraries, and anti-idiotypic (anti-Id) antibodies. Regardless of structure, an antibody fragment binds to the same antigen that is recognized by the intact antibody. The term "antibody fragment" encompasses DART molecules and diabodies. The term "antibody fragment" further includes any synthetic or genetically engineered protein that contains an immunoglobulin variable region and functions like an antibody by forming a complex through binding to a specific antigen. A "single-chain variable fragment" or "scFv" refers to a fusion protein of the variable regions of the heavy chain (VH) and light chain (VL) of an immunoglobulin. In certain aspects, the domains are connected by a short linker peptide of 10 to about 25 amino acids. The linker may be rich in glycine for flexibility as well as serine or threonine for solubility, and may connect the N-terminus of VH to the C-terminus of VL, or vice versa. Despite removal of the constant regions and introduction of the linker, such protein retains the specificity of the parent immunoglobulin. With regard to IgG, a standard immunoglobulin molecule comprises two identical light chain polypeptides with a molecular weight of approximately 23,000 daltons and two identical heavy chain polypeptides with a molecular weight ranging from 53,000 to 70,000 daltons. The four chains are generally connected via disulfide bonds in a "Y" configuration, where each light chain brackets a heavy chain starting from the mouth of the "Y" and extends through the variable region.

As described above, variable regions enable an antibody to selectively recognize and specifically bind an epitope on an antigen. That is, the VL and VH domains of an antibody, or subsets of the complementarity-determining regions (CDRs) of an antibody, associate to form a variable region that defines a three-dimensional antigen-binding site. This quaternary antibody structure forms the antigen-binding sites present at the terminus of each arm of the Y-shaped configuration. More specifically, the antigen-binding site is defined by three CDRs on each of the VH and VL chains (i.e., HCDR1, HCDR2, HCDR3, LCDR1, LCDR2 and LCDR3). In certain instances, for example, certain immunoglobulin molecules are derived from camelid species or engineered based on camelid immunoglobulins. Alternatively, an immunoglobulin molecule may consist of heavy chains only, with no light chains, or light chains only, with no heavy chains.

In naturally occurring antibodies, the six CDRs present within each antigen-binding domain are short, non-contiguous amino acid sequences that are specifically positioned to form the "antigen-binding domain" as the antibody assumes its three-dimensional conformation in an aqueous environment. The remaining amino acids within the antigen-binding domain, referred to as framework domains, exhibit relatively low inter-molecular variability. The framework regions mainly adopt β-sheet conformations, and the CDRs form loops which connect, and in some cases form part of, the β-sheet structures. The framework regions thus function as a scaffold to position the CDRs in the proper orientation via interchain noncovalent interactions. The antigen-binding domain formed by the positioned CDRs defines a surface complementary to the epitope on the immunoreactive antigen. This complementary surface promotes noncovalent binding of the antibody to its cognate epitope. Given the precise delineation available, a person of ordinary skill in the art can readily identify the amino acids constituting the CDRs and framework regions, respectively, for any given heavy or light chain variable region.

As used herein, the term "light chain constant region (CL)" encompasses the CL amino acid sequence derived from an antibody light chain (SEQ ID NO: 121 or 122). Preferably, the light chain constant region comprises at least one of a constant κ domain or a constant λ domain.

As used herein, the term "heavy chain constant region (CH)" encompasses amino acid sequences derived from an immunoglobulin heavy chain. A polypeptide comprising a heavy chain constant region contains at least one of the following: a CH1 domain (SEQ ID NO: 111 or 119), a hinge domain (e.g., upper, middle and/or lower hinge region), a CH2 domain, a CH3 domain, or variants or fragments thereof. It is understood that heavy chain constant regions may be modified such that their amino acid sequences differ from those of naturally occurring immunoglobulin molecules.

In one embodiment of the present invention, the prepared multispecific antibody comprises a CrossMab structure, in which partial amino acid sequences of the heavy chain CH1 and light chain CL are exchanged to prevent mispairing. Such a structure comprises CH1 as set forth in SEQ ID NO: 112 and CL as set forth in SEQ ID NO: 113.

As used herein, a "variant" of an antibody, antibody fragment or antibody domain refers to an antibody, antibody fragment or antibody domain that (1) shares at least 80%, 85%, 90%, 95%, 96%, 97%, 98% or 99% sequence identity with the parent antibody, antibody fragment or antibody domain, and (2) specifically binds to the same target as the parent antibody, antibody fragment or antibody domain. It is understood that where sequence identity is expressed as "at least x% identical" or "at least x% identity", such embodiments include any and all numerical percentages equal to or above the lower limit. Furthermore, it shall be understood that wherever an amino acid sequence is present in the present application, the disclosure is construed to additionally cover or encompass sequences having at least 80%, at least 85%, at least 90%, at least 95%, at least 96%, at least 97%, at least 98% or at least 99% identity to said amino acid sequence.

Various compositions and methods are encompassed within the scope of the multispecific molecules of the present invention, including: asymmetric IgG-like antibodies (e.g., trifunctional monoclonal antibodies/triomab/quadroma); knobs-into-holes antibodies; CrossMab; electrostatically matched antibodies; LUZ-Y; strand-exchange engineered domain (SEED) bodies; Fab-exchanged antibodies; symmetric IgG-like antibodies; two-in-one antibodies; cross-linked monoclonal antibodies, mAb²; CovX-body; dual-variable-domain (DVD)-Ig fusion proteins; IgG-like bispecific antibodies; Ts2Ab; BsAb; scFv/Fc fusions; bis(scFv)₂-Fabs; F(ab)₂ fusion proteins; dual-action or Bis-Fab; Dock-and-Lock (DNL); Fab-Fv; scFv-based antibodies and diabody-based antibodies (such as bispecific T-cell engagers (BiTEs); tandem diabodies (Tandab); DARTs; single-chain diabodies; TCR-like antibodies; human serum albumin-scFv fusion proteins, COMBODIES, and IgG/non-IgG fusion proteins.

As used herein, the phrase "multispecific antibody" refers to a molecule comprising at least two targeting domains with distinct binding specificities, wherein at least one targeting domain specifically binds CCR8, an antigen expressed on the surface of Treg cells. In certain embodiments, the multispecific antibody is a polypeptide comprising a scaffold and two or more immunoglobulin antigen-binding domains directed against different antigens or epitopes. In some embodiments, the multispecific antibody is a bispecific antibody. In other embodiments, the multispecific antibody is a trispecific antibody.

As used herein, the term "bispecific" refers to a molecule comprising at least two targeting domains possessing different binding specificities. Each targeting domain is capable of specifically binding to a target molecule and inhibiting the biological function of the target molecule upon binding. In some embodiments, the bispecific antibody is a polymeric molecule composed of two or more polypeptides. In certain embodiments, the targeting domain comprises an antigen-binding domain or CDRs of an antibody. In other embodiments, the targeting domain comprises a ligand or fragment thereof that specifically binds the target protein.

The terms "bispecific antibody", "bispecific molecule" and "bi-antibody" are used interchangeably herein and refer to an antibody capable of specifically binding two distinct antigens (or epitopes). In some embodiments, the bispecific antibody is a full-length antibody that binds one antigen (or epitope) on one of its two binding arms (one pair of HC/LC) and binds a different antigen (or epitope) on its second arm (the other pair of HC/LC). In such embodiments, the bispecific antibody has two distinct antigen-binding arms (in both specificity and CDR sequences) and is monovalent for each antigen it binds.

In other embodiments, the bispecific antibody is a full-length antibody capable of binding two different antigens (or epitopes) in each of its two binding arms (two pairs of HC/LC). In these embodiments, the bispecific antibody has two identical antigen-binding arms with identical specificity and identical CDR sequences and is bivalent for each antigen to which it binds.

The terms "trispecific antibody", "trispecific molecule" and "tri-antibody" are used interchangeably herein and refer to a molecule comprising three targeting domains with three distinct binding specificities. Each targeting domain is capable of specifically binding to a target molecule and inhibiting the biological function of the target molecule upon binding. In some embodiments, the trispecific antagonist is a polymeric molecule composed of two or more polypeptides. In certain embodiments, the targeting domain comprises an antigen-binding domain or CDRs of an antibody. In other embodiments, the targeting domain comprises a ligand or fragment thereof that specifically binds the target protein.

In a preferred embodiment of the present invention, bispecific antibodies targeting CCR8 and VEGF/PD-L1 were constructed, adopting the formats illustrated in panels a-i of FIG. 7A, and exemplary molecules corresponding to formats a-i are depicted in FIG. 7B-K. The anti-CCR8 Fab or anti-CCR8 scFv may be a Fab or scFv constructed using the VH and VL of any anti-CCR8 antibody disclosed herein; the anti-VEGF Fab or anti-VEGF scFv may be a Fab or scFv constructed using the VH and VL of any anti-VEGF antibody disclosed herein; and the anti-PD-L1 Fab or anti-PD-L1 scFv may be a Fab or scFv constructed using the VH and VL of any anti-PD-L1 antibody disclosed herein.

In another preferred embodiment of the present invention, trispecific antibodies targeting CCR8, VEGF and PD-L1 were constructed, adopting the formats illustrated in panels a-d of FIG. 8A, and exemplary molecules corresponding to formats a-d are depicted in FIG. 8B-E. The anti-CCR8 Fab may be a Fab constructed using the VH and VL of any anti-CCR8 antibody disclosed herein; the anti-VEGF Fab or anti-VEGF scFv may be a Fab or scFv constructed using the VH and VL of any anti-VEGF antibody disclosed herein; and the anti-PD-L1 scFv may be an scFv constructed using the VH and VL of any anti-PD-L1 antibody disclosed herein.

The present invention further provides polynucleotide molecules encoding the aforementioned antibodies or fragments thereof. The polynucleotides of the present invention may be in DNA or RNA form. DNA forms include cDNA, genomic DNA or synthetic DNA. The DNA may be single-stranded or double-stranded, and may be the coding strand or non-coding strand. The coding sequence encoding the mature polypeptide may be identical to the coding sequence of the antibody of the present invention or may be a degenerate variant. As used herein, a "degenerate variant" in the present invention refers to a nucleic acid sequence that encodes a polypeptide having the same amino acid sequence as the polypeptide of the present invention but possesses a different coding sequence.

Polynucleotides encoding the mature polypeptides of the present invention include: coding sequences encoding only the mature polypeptide; coding sequences for the mature polypeptide plus various additional coding sequences; coding sequences for the mature polypeptide (and optional additional coding sequences) together with non-coding sequences.

The term "polynucleotide encoding a polypeptide" may refer to a polynucleotide comprising the coding sequence for the polypeptide, or a polynucleotide further containing additional coding and/or non-coding sequences.

The present invention also relates to polynucleotides that hybridize with the above sequences and share at least 50%, preferably at least 70%, more preferably at least 80% identity between the two sequences. In particular, the present invention relates to polynucleotides capable of hybridizing to the polynucleotides disclosed herein under stringent conditions. As used herein, "stringent conditions" refer to: (1) hybridization and washing under low ionic strength and high temperature, e.g., 0.2×SSC, 0.1% SDS, at 60 °C; or (2) hybridization in the presence of a denaturant such as 50% (v/v) formamide, 0.1% bovine serum albumin / 0.1% Ficoll, at 42 °C; or (3) hybridization occurring only when sequence identity between two sequences is at least 90%, more preferably at least 95%.

The full-length nucleotide sequences encoding the antibodies of the present invention or fragments thereof can generally be obtained by PCR amplification, recombinant methods or chemical synthesis. One feasible approach is chemical synthesis of target sequences, especially for relatively short fragments. Typically, long sequences can be obtained by synthesizing multiple short fragments followed by ligation. In addition, the coding sequence of the heavy chain can be fused with an expression tag (such as 6×His) to form a fusion protein.

Once the relevant sequence is obtained, recombinant methods can be adopted to produce the sequence in large quantities. This generally involves cloning the sequence into a vector, transforming the vector into host cells, and isolating the target sequence from propagated host cells by conventional methods. Biological molecules (nucleic acids, proteins, etc.) involved in the present invention include such molecules in isolated form.

At present, DNA sequences encoding the proteins (or fragments or derivatives thereof) of the present invention can be fully obtained by chemical synthesis. Such DNA sequences can then be introduced into various existing DNA molecules (such as vectors) and cells known in the art. Furthermore, mutations can be introduced into the protein sequences of the present invention via chemical synthesis.

The present invention also relates to vectors containing the appropriate DNA sequences described above together with suitable promoters or regulatory sequences. Such vectors can be used to transform appropriate host cells to enable protein expression.

Host cells may be prokaryotic cells such as bacterial cells; lower eukaryotic cells such as yeast cells; or higher eukaryotic cells such as mammalian cells. Representative examples include: *Escherichia coli*, *Streptomyces*; bacterial cells of *Salmonella typhimurium*; fungal cells such as yeast; insect cells such as Drosophila S2 or Sf9; animal cells such as CHO and COS7 and HEK293 cells.

Transformation of host cells with recombinant DNA can be performed using conventional techniques well known to those skilled in the art. When the host is a prokaryote such as *E. coli*, competent cells capable of taking up DNA can be harvested after logarithmic growth phase and treated by the CaCl₂ method, and the relevant procedures are well known in the art. An alternative method employs MgCl₂. If desired, transformation may also be carried out by electroporation. Where the host is a eukaryote, suitable DNA transfection methods include calcium phosphate coprecipitation, conventional mechanical means such as microinjection, electroporation, liposome encapsulation and the like.

The resulting transformants can be cultured by conventional methods to express the polypeptide encoded by the gene of the present invention. Depending on the host cell employed, culture media can be selected from various conventional media. Cultivation is performed under conditions suitable for host cell growth. After host cells grow to an appropriate cell density, the selected promoter is induced by suitable means (such as temperature shift or chemical induction), and cells are cultured for an additional period.

The recombinant polypeptide produced in the above methods may be expressed intracellularly, on the cell membrane, or secreted extracellularly. If necessary, the recombinant protein can be isolated and purified by various separation methods taking advantage of its physical, chemical and other properties. Such methods are well known to those skilled in the art. Examples include, but are not limited to: conventional renaturation treatment, treatment with protein precipitants (salting-out), centrifugation, osmotic cell lysis, ultrasonication, ultracentrifugation, molecular sieve chromatography (gel filtration), adsorption chromatography, ion exchange chromatography, high-performance liquid chromatography (HPLC), other liquid chromatographic techniques, and combinations thereof.

The antibodies of the present invention can be used alone, or conjugated or coupled with detectable labels (for diagnostic purposes), therapeutic agents, PK (protein kinase)-modifying moieties, or any combination of the foregoing substances.

Detectable labels for diagnostic purposes include, but are not limited to: fluorescent or luminescent labels, radioactive labels, MRI (magnetic resonance imaging) or CT (computed tomography) contrast agents, or enzymes capable of generating detectable products.

Therapeutic agents that may be conjugated include, but are not limited to: insulin, IL-2, interferons, calcitonin, GHRH peptides, intestinal peptide analogues, albumin, antibody fragments, cytokines and hormones.

The present invention also provides a composition. In a preferred embodiment, the composition is a pharmaceutical composition comprising the aforementioned antibody or active fragment thereof or fusion protein, together with a pharmaceutically acceptable carrier. Typically, such materials can be formulated in a non-toxic, inert, pharmaceutically acceptable aqueous carrier medium, wherein the pH is generally about 5-8, preferably about 6-8, although the pH may vary depending on the nature of the formulated substance and the disorder to be treated. The formulated pharmaceutical composition may be administered via conventional routes including, but not limited to: oral, respiratory, intratumoral, intraperitoneal, intravenous, or local administration.

The pharmaceutical composition of the present invention can be used for treating cancers/tumors, especially solid tumors, in particular solid tumors with high expression of CCR8.

The pharmaceutical composition of the present invention contains a safe and effective amount (e.g., 0.001-99 wt%, preferably 0.01-90 wt%, more preferably 0.1-80 wt%) of the aforementioned monoclonal antibody (or conjugate thereof) of the present invention, together with a pharmaceutically acceptable carrier or excipient. Such carriers include, but are not limited to: saline, buffer solutions, glucose, water, glycerol, ethanol, and combinations thereof. The pharmaceutical formulation should be compatible with the mode of administration. The pharmaceutical composition of the present invention can be prepared in injectable form, for example, by conventional methods using normal saline or aqueous solutions containing glucose and other adjuvants. Pharmaceutical compositions such as injections and solutions are preferably manufactured under sterile conditions. The active ingredient is administered at a therapeutically effective dose, for example, approximately 1 µg/kg body weight to approximately 10 mg/kg body weight per day. In addition, the pharmaceutical composition of the present invention may be used in combination with other therapeutic agents.

When the pharmaceutical composition is used, a safe and effective amount of the immunoconjugate is administered to a mammal. The safe and effective amount is generally at least about 10 µg/kg body weight, and in most cases does not exceed about 8 mg/kg body weight; preferably, the dosage ranges from about 10 µg/kg body weight to about 1 mg/kg body weight. Naturally, the specific dosage should also take into account factors such as administration route and patient health status, all of which fall within the skill of qualified physicians.

The present invention will be further explained in conjunction with specific examples. It should be understood that these examples are only used to illustrate the present invention and not to limit the scope of the present invention. The experimental methods without specific conditions specified in the following examples are usually carried out under conventional conditions, such as those described in Sambrook et al., Molecular Cloning: Laboratory Manual (New York: Cold Spring Harbor Laboratory Press, 1989), or according to the conditions recommended by the manufacturer. Unless otherwise specified, percentages and parts are calculated by weight.

### Sequences Used for Constructing the Multispecific Antibodies of the Present Invention Anti-CCR8 Antibody Sequences

| | HCDR1 | HCDR2 | HCDR3 |
|---|---|---|---|
| C61H | GFIFSNAWMT (SEQ ID NO: 1) | RIKRKSDGGTTDYAASVK G (SEQ ID NO: 2) | PTMVRGVLFDY (SEQ ID NO: 3) |
| C5H | GFTFSNYGMH (SEQ ID NO: 4) | VIWYDGSKKYYADSVKG (SEQ ID NO: 5) | GPPRYDWKYGYFDY (SEQ ID NO: 6) |
| C6H | GFTFSNAWMS (SEQ ID NO: 7) | RIKRKTDGGTRDYAAPV KG (SEQ ID NO: 8) | ITMVRGVVFDY (SEQ ID NO: 9) |
| C20H | GFIFSNAWMS (SEQ ID NO: 10) | RIKRKSDGGTTDYAAPVR G (SEQ ID NO: 11) | ITLVRGVLFDY (SEQ ID NO: 12) |
| C23H | GFTFSNAWMS (SEQ ID NO: 7) | RIKRKSDGGTTDYAAPVK G (SEQ ID NO: 13) | ITLTRGVLFDY (SEQ ID NO: 14) |
| C24H | GFTFSSYGMH (SEQ ID NO: 15) | AISYDGNIKHYADSVKG (SEQ ID NO: 16) | TGLAY (SEQ ID NO: 17) |
| C27H | GFTFSSYGIH (SEQ ID NO: 18) | VIWYDGSKKYYADSVKG (SEQ ID NO: 5) | GPPRYNWNYGYFDY (SEQ ID NO: 19) |
| C39H | GYTFTSYALH (SEQ ID NO: 20) | WIKGDNGNTKYSQKFQG (SEQ ID NO: 21) | SGYSYGYAFDI (SEQ ID NO: 22) |
| C40H | GFTFSNAWMS (SEQ ID NO: 7) | RIKRKTDGGTTDYAAPVKG (SEQ ID NO: 23) | ITMVRGVVFDY (SEQ ID NO: 9) |
| C46H | GFIFSNAWMI (SEQ ID NO: 24) | RIKSKSDGGTRDYAGPVK G (SEQ ID NO: 25) | ITMVRGVLFDY (SEQ ID NO: 26) |
| C53H | GFTFSNAWMS (SEQ ID NO: 7) | RIKRKSDGGTTDYAAPVK G (SEQ ID NO: 13) | ITLTRGVLSDY (SEQ ID NO: 27) |
| C54H | GFTFSNAWMT (SEQ ID NO: 28) | RIKRKSDGGTTDYAASVK G (SEQ ID NO: 2) | VTMVRGVLFDY (SEQ ID NO: 29) |
| C57H | SGSISSSNWW (SEQ ID NO: 30) | GVYHSGGTNYNPSLKS (SEQ ID NO: 31) | EKRITMVRGVIINGGFD Y (SEQ ID NO: 32) |
| C62H | GFTFSNAWMS (SEQ ID NO: 7) | RIKRKSDGGTTDYAAPVK G (SEQ ID NO: 13) | ITLTRGVLFDY (SEQ ID NO: 14) |
| | LCDR1 | LCDR2 | LCDR3 |
| C61L | RASQSVSSGSLA (SEQ ID NO: 33) | GASSRTT (SEQ ID NO: 34) | QQYGSSRT (SEQ ID NO: 35) |
| C5L | RASQGIRNHLG (SEQ ID NO: 36) | AASSLQS (SEQ ID NO: 37) | LQHNSYLT (SEQ ID NO: 38) |
| C6L | RASQTISINYLA (SEQ ID NO: 39) | GASSRVT (SEQ ID NO: 40) | QQYGSSRT (SEQ ID NO: 35) |
| C20L | RASQSVNINLA (SEQ ID NO: 41) | GASSRTT (SEQ ID NO: 34) | QQYGGSRT (SEQ ID NO: 42) |
| C23L | RASQSVSSGSLA (SEQ ID NO: 33) | GASSRTT (SEQ ID NO: 34) | QQYGSSRT (SEQ ID NO: 35) |
| C24L | RSSQSLVYSDGNTY LT (SEQ ID NO: 43) | KVSNRDS (SEQ ID NO: 44) | MHNTNWPIT (SEQ ID NO: 45) |
| C27L | RASQGIRNDLG (SEQ ID NO: 46) | AASSLQS (SEQ ID NO: 34) | LQHNSYLT (SEQ ID NO: 38) |
| C39L | RASQGIRNNLG (SEQ ID NO: 47) | GASSLQS (SEQ ID NO: 48) | LQHNSYPPT (SEQ ID NO: 49) |
| C40L | RASQTISINYLA (SEQ ID NO: 39) | GASSRVT (SEQ ID NO: 40) | QQYGSSRT (SEQ ID NO: 35) |
| C46L | RASQSISSSSLA (SEQ ID NO: 50) | GVSSRAA (SEQ ID NO: 51) | QQYGVSRT (SEQ ID NO: 52) |
| C53L | RASQSVSSGSLA (SEQ ID NO: 33) | GASSRAT (SEQ ID NO: 53) | QQYGSSRT (SEQ ID NO: 35) |
| C54L | RASQSVSSGSLA (SEQ ID NO: 33) | GASSRAT (SEQ ID NO: 53) | QQYGTSRT (SEQ ID NO: 54) |
| C57L | RASQSISRWLA (SEQ ID NO: 55) | KASSLES (SEQ ID NO: 56) | QQYSSYSWT (SEQ ID NO: 57) |
| C62L | RASQSVSSGSLA (SEQ ID NO: 33) | SASSRAT (SEQ ID NO: 58) | QQYGSSRT (SEQ ID NO: 35) |

### Anti-CCR8 Antibody Sequences

| | |
|---|---|
| EVQLVESGGGLVKPGGSLRLSCAASGFIFSNAWMTWVRQAPGKGLEW VGRIKRKSDGGTTDYAASVKGRFTISRDDSKNTLYLQMNSLKTEDAAV YYCSTPTMVRGVLFDYWGQGTLVTVSS | VH; C61H (SEQ ID NO: 59) |
| QVQLVESGGGVVQPGRSLRLSCVASGFTFSNYGMHWVRQAPGRGLEW VAVIWYDGSKKYYADSVKGRFTISRDNSKNTLFLQMNSLRAEDTAVYY CARGPPRYDWKYGYFDYWGQGTLVTVSS | VH; C5H (SEQ ID NO: 60) |
| EVHLVESGGGLVKPGGSLRLSCEASGFTFSNAWMSWVRQAPGKGLEW VGRIKRKTDGGTRDYAAPVKGRFTISRDDSKNTLYLQMNSLKTEDTAV YYCTTITMVRGVVFDYWGQGTLVTVSS | VH; C6H (SEQ ID NO: 61) |
| EVQLVESGGGLVKPGGSLRLSCAGSGFIFSNAWMSWVRQAPGKGLEWI GRIKRKSDGGTTDYAAPVRGRFAISRDDSKNTLYLQISSLKTEDTAVYY CTTITLVRGVLFDYWGQGTLVTVSS | VH; C20H (SEQ ID NO: 62) |
| EVQLVESGGGLVKPGGSLRLSCAASGFTFSNAWMSWVRQAPGKRLEW VGRIKRKSDGGTTDYAAPVKGRFTISRDDSKNTLYLQMDSLKTEDTAV YYCTTITLTRGVLFDYWGQGTLVTVSS | VH; C23H (SEQ ID NO: 63) |
| QVQLVESGGGVVQPGRSLRLSCAASGFTFSSYGMHWVRQAPGTGLEW VAAISYDGNIKHYADSVKGRFTISRDNSKNTLYLQMSSLRPEDTAVYYC AKTGLAYWGQGTLVTVSS | VH; C24H (SEQ ID NO: 64) |
| QVQLVESGGGVVQPGRSLRLSCAASGFTFSSYGIHWVRQAPGKGLEW VAVIWYDGSKKYYADSVKGRFTISRDNSKNTLYLQMNSLRADDTAVY YCARGPPRYNWNYGYFDYWGQGTLVTVSS | VH; C27H (SEQ ID NO: 65) |
| QVQVVQSGAEVKKPGASVTVSCKASGYTFTSYALHWVRQAPGQRLEC MGWIKGDNGNTKYSQKFQGRVTFTRDTSASTAYMELSSLRSEDTAVY YCARSGYSYGYAFDIWGQGTMVTVSS | VH; C39H (SEQ ID NO: 66) |
| EVRLVESGGGLVKPGGSLRLSCEASGFTFSNAWMSWVRQAPGKGLEW VGRIKRKTDGGTTDYAAPVKGRFTISRDDSKSTLYLQMNTLKTEDTAV YYCSTITMVRGVVFDYWGQGTLVTVSS | VH; C40H (SEQ ID NO: 67) |
| EVQLVESGGGLVKPGGSLRLSCTASGFIFSNAWMIWVRQAPGKGLEWV GRIKSKSDGGTRDYAGPVKGRFTISRDDSKKMVYLQMNSLKTEDTAV YYCSTITMVRGVLFDYWGQGTLVTVSS | VH; C46H (SEQ ID NO: 68) |
| EVQLVESGGGLVKPGGSLRLSCAASGFTFSNAWMSWVRQAPGKGLEW VGRIKRKSDGGTTDYAAPVKGRFTISRDDSRNTLYLQMNSLKNEDTAV YYCTTITLTRGVLSDYWGQGTLVTVSS | VH; C53H (SEQ ID NO: 69) |
| EVQLVESGGGLVKPGGSLRLSCTASGFTFSNAWMTWVRQAPGKGLEW VGRIKRKSDGGTTDYAASVKGRFTISRDDSKNTLYLQMNSLKTEDAAV YYCSTVTMVRGVLFDYWGQGTLVTVSS | VH; C54H (SEQ ID NO: 70) |
| QVQLQESGPGLVKPSGTLSLACAVSSGSISSSNWWSWVRQPPGKGLEW IGGVYHSGGTNYNPSLKSRVTISVDKSKNLFSLKLSSVTAADTAVYYCA REKRITMVRGVIINGGFDYWGQGTLVTVSS | VH; C57H (SEQ ID NO: 71) |
| EVQLVESGGGLVKPGGSLRLSCAASGFTFSNAWMSWVRQAPGKGLEW VGRIKRKSDGGTTDYAAPVKGRFTISRDDSKNTLFLQMNSLKTEDTAV YYCTTITLTRGVLFDYWGQGTLVTVSS | VH; C62H (SEQ ID NO: 72) |
| EIVLTQSPGTLSLSPGERATLSCRASQSVSSGSLAWYQQKPGQAPRLLIY GASSRTTGIPDRFSGSGSGTDFTLTITRLEPEDFAVYYCQQYGSSRTFGQ GTRVEIK | VL; C61L (SEQ ID NO: 73) |
| DIQMTQSPSSLSASVGDRVTITCRASQGIRNHLGWYQQKPGKAPKRLIY AASSLQSGVPSRFSGSGSGTEFTLTISSLQPEDFATYYCLQHNSYLTFGG GTKVEIK | VL; C5L (SEQ ID NO: 74) |
| EIVLTQSPGTLSLSPGERAALSCRASQTISINYLAWYQQKPGQAPRLLIY GASSRVTGIPDRFSGSGSGTDFTLTISRLEPEDFAVYYCQQYGSSRTFGQ GTKVEFK | VL; C6L (SEQ ID NO: 75) |
| EIVLTQSPGTLSLSPGERATLSCRASQSVNINLAWYQQKPGQAPRLLIYG ASSRTTGIPDRFSGSGSGTDFSLTISRLEPEDFAVYYCQQYGGSRTFGQG TKVEIK | VL; C20L (SEQ ID NO: 76) |
| EVVLTQSPGTLSLSPGERATLSCRASQSVSSGSLAWYQQKPGQAPRLLI YGASSRTTGIPDRFSGSGSGTDFTLTISRLESEDFALYYCQQYGSSRTFG QGTKVEIK | VL; C23L (SEQ ID NO: 77) |
| DIVMTQSPLSLPVTLGQPASISCRSSQSLVYSDGNTYLTWFQQRPGQSPR RLIYKVSNRDSGVADRFSGSGSGTDFTLKISRVEAEDVGVYYCMHNTN WPITFGQGTRLEIK | VL; C24L (SEQ ID NO: 78) |
| DIQMTQSPSSLSASVGDRVTITCRASQGIRNDLGWYQQKPGKAPKRLIY AASSLQSGVPSRFSGSGSGTEFTLTISSLQPEDFATYYCLQHNSYLTFGG GTKVEIK | VL; C27L (SEQ ID NO: 79) |
| DIQMTQSPSSLSASVRDRVTLTCRASQGIRNNLGWYQQKPGKAPKRLI YGASSLQSGVPSRFSGSGSGTEFTLTISSLQPEDFATYYCLQHNSYPPTF GQGTQVEIK | VL; C39L (SEQ ID NO: 80) |
| QIVLTQSPGTLSLSPGERAALSCRASQTISINYLAWYQQKPGQAPRLLIY GASSRVTGIPDRFSGSGSGTDFTLTISRLEPEDFAVYYCQQYGSSRTFGQ GTKVEFK | VL; C40L (SEQ ID NO: 81) |
| EIVLTQSPGTLSLSPGERATLSCRASQSISSSSLAWYQQTPGQTPRLLIYG VSSRAAGIPDRFSGSGSGTDFTLTISRLEPEDFAVYYCQQYGVSRTFGQG TKVEIK | VL; C46L (SEQ ID NO: 82) |
| EVVLTQSPGTLSLSPGERATLSCRASQSVSSGSLAWYQQKPGQAPRLLI YGASSRATGIPDRFSGSGSGTDFTLTISRLESEDFALYYCQQYGSSRTFG QGTKVEIK | VL; C53L (SEQ ID NO: 83) |
| EIVLTQSPGTLSLSPGERATLSCRASQSVSSGSLAWYQQKPGQAPRLFIY GASSRATGVPDRFSGSGSGTDFTLTISRLEPEDFAIYYCQQYGTSRTFGQ GTRVEFK | VL; C54L (SEQ ID NO: 84) |
| DIQMTQSPSTLSASVGDRVTITCRASQSISRWLAWYRQKPGKAPKLLIY KASSLESGVPSRFSGSGSGTEFTLTISSLQPDDFASYYCQQYSSYSWTFG QGTKVEIK | VL; C57L (SEQ ID NO: 85) |
| EVVLTQSPGTLSLSPGERATLSCRASQSVSSGSLAWYQQKPGQAPRLLI YSASSRATGIPDRFSGSGSGTDFTLTISRLESEDFALYYCQQYGSSRTFG QGTKVEIK | VL; C62L (SEQ ID NO: 86) |

### Anti-VEGF Antibody Sequences

| | |
|---|---|
| | VH; AVAH (SEQ ID NO: 87) |
| EVQLVESGGGLVQPGGSLRLSCAASGYTFTNYGMNWVRQAPGKCL EWVGWINTYTGEPTYAADFKRRFTFSLDTSKSTAYLQMNSLRAEDT AVYYCAKYPHYYGSSHWYFDVWGQGTLVTVSS | VH; AVACH (SEQ ID NO: 88) |
| EVQLVESGGGLVQPGGSLRLSCAASGFTISDYWIHWVRQAPGKGLE WVAGITPAGGYTYYADSVKGRFTISADTSKNTAYLQMNSLRAEDTAV YYCARFVFFLPYAMDYWGQGTLVTVSS | VH; ASH (SEQ ID NO: 89) |
| EVQLVESGGGLVQPGGSLRLSCAASGFTISDYWIHWVRQAPGKCLE WVAGITPAGGYTYYADSVKGRFTISADTSKNTAYLQMNSLRAEDTAV YYCARFVFFLPYAMDYWGQGTLVTVSS | VH; ASCH (SEQ ID NO: 90) |
| EVQLVESGGGLVQPGGSLRLSCAASGFTIDDYWIHWVRQAPGKGLE WVAGITPAGGYTYYADSVKGRFTISADTSKNTAYLQMNSLRAEDTAV YYCARFVFFLPYAMDYWGQGTLVTVSS | VH; AS2H (SEQ ID NO: 91) |
| EVQLVESGGGLVQPGGSLRLSCAASGFTIDDYWIHWVRQAPGKCLE WVAGITPAGGYTYYADSVKGRFTISADTSKNTAYLQMNSLRAEDTAV | VH; AS2CH (SEQ ID NO: 92) |
| YYCARFVFFLPYAMDYWGQGTLVTVSS | |
| EVQLVESGGGLVQPGGSLRLSCAASGFTIEDYWIHWVRQAPGKGLE WVAGITPAGGYTYYADSVKGRFTISADTSKNTAYLQMNSLRAEDTAV YYCARFVFFLPYAMDYWGQGTLVTVSS | VH; AS3H (SEQ ID NO: 149) |
| EVQLVESGGGLVQPGGSLRLSCAASGFTIEDYWIHWVRQAPGKCLE WVAGITPAGGYTYYADSVKGRFTISADTSKNTAYLQMNSLRAEDTAV YYCARFVFFLPYAMDYWGQGTLVTVSS | VH; AS3CH (SEQ ID NO: 150) |
| | VL; AVAL (SEQ ID NO: 93) |
| DIQMTQSPSSLSASVGDRVTITCSASQDISNYLNWYQQKPGKAPKVL IYFTSSLHSGVPSRFSGSGSGTDFTLTISSLQPEDFATYYCQQYSTVPW TFGCGTKVEIK | VL; AVACL (SEQ ID NO: 94) |
| DIQMTQSPSSLSASVGDRVTITCRASQDVSTAVAWYQQKPGKAPKLL IYSASFLYSGVPSRFSGSGSGTDFTLTISSLQPEDFATYYCQQGYGNPF TFGQGTKVEIK | VL; ASL (SEQ ID NO: 95) |
| DIQMTQSPSSLSASVGDRVTITCRASQDVSTAVAWYQQKPGKAPKLL IYSASFLYSGVPSRFSGSGSGTDFTLTISSLQPEDFATYYCQQGYGNPF TFGCGTKVEIK | VL; ASCL (SEQ ID NO: 96) |
| DIQMTQSPSSLSASVGDRVTITCRASQDVSTAVAWYQQKPGKAPKLL IYDASFLYSGVPSRFSGSGSGTDFTLTISSLQPEDFATYYCQQGYGNPF TFGQGTKVEIK | VL; AS2L (SEQ ID NO: 97) |
| DIQMTQSPSSLSASVGDRVTITCRASQDVSTAVAWYQQKPGKAPKLL IYDASFLYSGVPSRFSGSGSGTDFTLTISSLQPEDFATYYCQQGYGNPF TFGCGTKVEIK | VL; AS2CL (SEQ ID NO: 98) |
| DIQMTQSPSSLSASVGDRVTITCRASQDVSTAVAWYQQKPGKAPKLL IYSADFLYSGVPSRFSGSGSGTDFTLTISSLQPEDFATYYCQQGYGNPF TFGQGTKVEIK | VL; AS3L (SEQ ID NO: 99) |
| DIQMTQSPSSLSASVGDRVTITCRASQDVSTAVAWYQQKPGKAPKLL IYSADFLYSGVPSRFSGSGSGTDFTLTISSLQPEDFATYYCQQGYGNPF TFGCGTKVEIK | VL; AS3CL (SEQ ID NO: 100) |
| DIQMTQSPSSLSASVGDRVTITCRASQDVSTAVAWYQQKPGKAPKLL IYDADFLYSGVPSRFSGSGSGTDFTLTISSLQPEDFATYYCQQGYGNP FTFGQGTKVEIK | VL; AS4L (SEQ ID NO: 101) |
| DIQMTQSPSSLSASVGDRVTITCRASQDVSTAVAWYQQKPGKAPKLL IYDADFLYSGVPSRFSGSGSGTDFTLTISSLQPEDFATYYCQQGYGNP FTFGCGTKVEIK | VL; AS4CL (SEQ ID NO: 102) |

Among them, AVACH and AVACL are variants of the heavy chain variable region AVAH and light chain variable region AVAL of antibody molecule AVA, respectively. The heavy chain variable region ASH and light chain variable region ASL of antibody molecule AS-1 are derived from U.S. Patent No. US7758859. ASCH, AS2H, AS2CH, AS3H and AS3CH are variants of its VH; ASCL, AS2L, AS2CL, AS3L, AS3CL, AS4L and AS4CL are variants of its VL. The underlined segments correspond sequentially to CDR1, CDR2 and CDR3 of the heavy chain variable region or light chain variable region, and mutation sites are indicated in **bold**.

### Anti-PD-L1 Antibody Sequences

| | |
|---|---|
| | VH;PL1H (SEQ ID NO: 103) |
| EVQLVESGGGLVQPGGSLRLSCAASGFTFSDSWIHWVRQAPGKCL EWVAWISPYGGSTYYADSVKGRFTISADTSKNTAYLQMNSLRAED TAVYYCARRHWPGGFDYWGQGTLVTVSS | VH;PL1CH (SEQ ID NO: 104) |
| EVQLLESGGGLVQPGGSLRLSCAASGFTFSSYIMMWVRQAPGKGL EWVSSIYPSGGITFYADTVKGRFTISRDNSKNTLYLQMNSLRAEDT AVYYCARIKLGTVTTVDYWGQGTLVTVSS | VH;PL2H (SEQ ID NO: 105) |
| EVQLLESGGGLVQPGGSLRLSCAASGFTFSSYIMMWVRQAPGKCL EWVSSIYPSGGITFYADTVKGRFTISRDNSKNTLYLQMNSLRAEDT AVYYCARIKLGTVTTVDYWGQGTLVTVSS | VH;PL2CH (SEQ ID NO: 106) |
| | VL;PL1L (SEQ ID NO: 107) |
| DIQMTQSPSSLSASVGDRVTITCRASQDVSTAVAWYQQKPGKAPKL LIYSASFLYSGVPSRFSGSGSGTDFTLTISSLQPEDFATYYCQQYLYH PATFGCGTKVEIK | VL;PL1CL (SEQ ID NO: 108) |
| QSALTQPASVSGSPGQSITISCTGTSSDVGGYNYVSWYQQHPGKAP KLMIYDVSNRPSGVSNRFSGSKSGNTASLTISGLQAEDEADYYCSS YTSSSTRVFGTGTKVTVL | VL;PL2L (SEQ ID NO: 109) |
| QSALTQPASVSGSPGQSITISCTGTSSDVGGYNYVSWYQQHPGKAP KLMIYDVSNRPSGVSNRFSGSKSGNTASLTISGLQAEDEADYYCSS YTSSSTRVFGCGTKVTVL | VL;PL2CL (SEQ ID NO: 110) |

Among them, PL1CH and PL1CL are variants of the heavy chain variable region PL1H and light chain variable region PL1L of antibody molecule PL1, respectively; PL2CH and PL2CL are variants of the heavy chain variable region PL2H and light chain variable region PL2L of antibody molecule PL2, respectively.

### IgG1 CH Sequences

CH1 sequences are shown in below:

| | |
|---|---|
| ASTKGPSVFPLAPSSKSTSGGTAALGCLVKDYFPEPVTVSWNSG ALTSGVHTFPAVLQSSGLYSLSSVVTVPSSSLGTQTYICNVNHK PSNTKVDKRV | IgG1 CH1(SEQ ID NO: 111) |
| ASVAAPSVFIFPPSDEQLKSGTASVVCLLNNFYPREAKVQWKV DNALQSGNSQESVTEQDSKDSTYSLSSTLTLSKADYEKHKVYA CEVTHQGLSSPVTKSFNRGE | IgG1 CH1-CL Cross (SEQ ID NO: 112) |

Fc region sequences are shown in below:

| | |
|---|---|
| EPKSCDKTHTCPPCPAPELLGGPSVFLFPPKPKDTLMISRTPEVT CVVVDVSHEDPEVKFNWYVDGVEVHNAKTKPREEQYNSTYR VVSVLTVLHQDWLNGKEYKCKVSNKALPAPIEKTISKAKGQPR EPQVYTLPPSRDELTKNQVSLTCLVKGFYPSDIAVEWESNGQPE | IgG1 Fc(SEQ ID NO: 113) |
| NNYKTTPPVLDSDGSFFLYSKLTVDKSRWQQGNVFSCSVMHE ALHNHYTQKSLSLSPGK | |
| CDKTHTCPPCPAPELLGGPSVFLFPPKPKDTLMISRTPEVTCVV VDVSHEDPEVKFNWYVDGVEVHNAKTKPREEQYNSTYRVVS VLTVLHQDWLNGKEYKCKVSNKALPAPIEKTISKAKGQPREPQ VYTLPPSRDELTKNQVSLTCLVKGFYPSDIAVEWESNGQPENN YKTTPPVLDSDGSFFLYSKLTVDKSRWQQGNVFSCSVMHEAL HNHYTQKSLSLSPGK | IgG1 Fc(SEQ ID NO: 114) |

The Fc region may comprise the following mutations:

| The KIH or charged pairs mutations |
|---|
| Y349C/K370E/K409D/K439E, S354C/D356K/E357K/D399K; |
| S354C/T366W, Y349C/T366S/L368A/Y407V; |

In a preferred embodiment of the present invention, the Fc region has the following sequence:

| | |
|---|---|
| EPKSCDKTHTCPPCPAPELLGGPSVFLFPPKPKDTLMISRTPEVT CVVVDVSHEDPEVKFNWYVDGVEVHNAKTKPREEQYNSTYR VVSVLTVLHQDWLNGKEYKCKVSNKALPAPIEKTISKAKGQPR EPQVYTLPPCRKKLTKNQVSLTCLVKGFYPSDIAVEWESNGQP ENNYKTTPPVLKSDGSFFLYSKLTVDKSRWQQGNVFSCSVMH EALHNHYTQKSLSLSPGK | Fc(S354C/D356K/ E357K/D399K) (SEQ ID NO: 115) |
| CDKTHTCPPCPAPELLGGPSVFLFPPKPKDTLMISRTPEVTCVV VDVSHEDPEVKFNWYVDGVEVHNAKTKPREEQYNSTYRVVS VLTVLHQDWLNGKEYKCKVSNKALPAPIEKTISKAKGQPREPQ VYTLPPCRKKLTKNQVSLTCLVKGFYPSDIAVEWESNGQPENN YKTTPPVLKSDGSFFLYSKLTVDKSRWQQGNVFSCSVMHEAL HNHYTQKSLSLSPGK | Fc(S354C/D356K/ E357K/D399K) (SEQ ID NO: 116) |
| CDKTHTCPPCPAPELLGGPSVFLFPPKPKDTLMISRTPEVTCVV VDVSHEDPEVKFNWYVDGVEVHNAKTKPREEQYNSTYRVVS VLTVLHQDWLNGKEYKCKVSNKALPAPIEKTISKAKGQPREPQ VCTLPPSRDELTKNQVSLTCLVEGFYPSDIAVEWESNGQPENN YKTTPPVLDSDGSFFLYSDLTVDKSRWQQGNVFSCSVMHEAL HNHYTQESLSLSPGK | Fc(Y349C/K370E /K409D/K439E) (SEQ ID NO: 117) |
| EPKSCDKTHTCPPCPAPELLGGPSVFLFPPKPKDTLMISRTPEVT CVVVDVSHEDPEVKFNWYVDGVEVHNAKTKPREEQYNSTYR VVSVLTVLHQDWLNGKEYKCKVSNKALPAPIEKTISKAKGQPR EPQVCTLPPSRDELTKNQVSLTCLVEGFYPSDIAVEWESNGQPE NNYKTTPPVLDSDGSFFLYSDLTVDKSRWQQGNVFSCSVMHE ALHNHYTQESLSLSPGK | Fc(Y349C/K370E /K409D/K439E) (SEQ ID NO: 118) |

In another preferred embodiment of the present invention, the C-terminal lysine (K) of the aforementioned Fc region is truncated to reduce the formation of antibody charge isoforms.

### IgG4 CH Sequences

| | |
|---|---|
| ASTKGPSVFPLAPCSRSTSESTAALGCLVKDYFPEPVTVSWNSG ALTSGVHTFPAVLQSSGLYSLSSVVTVPSSSLGTKTYTCNVDHK PSNTKVDKRV | IgG4 CH1(SEQ ID NO: 119) |
| ESKYGPPCPPCPAPEFLGGPSVFLFPPKPKDTLMISRTPEVTCVV VDVSQEDPEVQFNWYVDGVEVHNAKTKPREEQFNSTYRVVSV | IgG4 Fc(S228P) (SEQ ID NO: 120) |
| LTVLHQDWLNGKEYKCKVSNKGLPSSIEKTISKAKGQPREPQV YTLPPSQEEMTKNQVSLTCLVKGFYPSDIAVEWESNGQPENNY KTTPPVLDSDGSFFLYSRLTVDKSRWQEGNVFSCSVMHEALHN HYTQKSLSLSLGK | |

The Fc region may comprise the following mutations:

| Mutations of the KIH or the charged pairs |
|---|
| Y349C/K370E/R409D/K439E, S354C/E356K/E357K/D399K, S354C/T366W, Y349C/T366S/L368A/Y407V |

### CL Sequences

| | |
|---|---|
| | CL; Kappa (SEQ ID NO: 121) |
| GQPKAAPSVTLFPPSSEELQANKATLVCLISDFYPGAVTVAWKA DSSPVKAGVETTTPSKQSNNKYAASSYLSLTPEQWKSHRSYSCQ VTHEGSTVEKTVAPTECS | CL; Lambda (SEQ ID NO: 122) |
| ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKKVEPKSC | CL-CH1 Cross (SEQ ID NO: 123) |

### Optional Linker Sequences

| |
|---|
| G |
| GS |
| SS |
| GSS |
| GSSSG |
| GGGGS |
| GGGGSGGGGS |
| GGGGSGGGGSGGGGS |
| GGGGSGGGGSGGGGSGGGGS |
| GGGGSGGGGSGGGGSGGGGSGGGGS |
| PGGGGSP |
| PGGGGSPGGGGSPGGGGSP |
| GEPGSGE |
| GEPGSGEGEPGSGE |
| GEPGSGEGEPGSGEEGEPGSGE |
| EGEPGSGEEGEPGSGEEGEPGSGEEGEPGSGE |
| GKPGS |
| GKPGSGKPGS |
| GKPGSGKPGSGKPGS |
| GKPGSGKPGSGKPGSGKPGS |
| GKPGSGKPGSGKPGSGKPGSGKPGS |
| SSSSG |
| SSSSGSSSSG |
| SSSSGSSSSGSSSSG |
| SSSSGSSSSGSSSSGSSSSG |
| GRPGSGPGSGRPGSGRPGS |
| GRPGSGPGSGRPGSGRPGSGRGPS |
| GKPGSGRPGSGKGPSGRPGS |

### Example 1 Preparation and Characterization of Anti-CCR8 Monoclonal Antibodies

### 1.1 Screening of Anti-CCR8 Monoclonal Antibodies

The anti-CCR8 antibodies of the present invention were obtained by hybridoma screening using humanized mice after immunization. A hCCR8 expression plasmid was constructed; DNA was extracted and endotoxin-free treated, and humanized mice with human heavy and light chain variable regions (disclosed in CN114763558A) were immunized. Spleens were harvested from immunized mice, and splenocytes were isolated and fused with Sp2/0-Ag14 myeloma cells. Cell fusion was performed by electroporation according to standard hybridoma fusion protocols. After fusion, a defined number of cells were seeded into each well of a 96-well plate and cultured in HAT medium for 10 days. Hybridoma culture supernatants were screened using a HEK293 cell line overexpressing human CCR8 to identify positive hybridoma clones. Positive hybridoma cells were cultured, and supernatants collected after 5 days of cell culture were subjected to small-scale antibody purification using Protein A magnetic beads to obtain hybridoma antibodies bearing human variable regions and murine constant regions. The purified antibodies were subjected to binding activity assays. During screening, specific binding molecules were identified against the extracellular domain of CCR8 or CCR8-overexpressing cell lines via ELISA and FACS. Finally, fourteen monoclonal antibodies were obtained (C5, C6, C20, C23, C24, C27, C39, C40, C46, C53, C54, C57, C61 and C62). Sequence analysis classified these fourteen antibodies into five groups. Among them, C24, C39 and C57 each form an independent group; C5 and C27 belong to one group; and the remaining antibodies fall into another group.

### 1.2 Binding Capacity of Anti-CCR8 Monoclonal Antibodies to CCR8-expressing HEK Cells

Further, the binding capacity of the anti-CCR8 antibodies of the present invention to HEK293 cells expressing CCR8 was evaluated. The CCR8-expressing HEK293 cell lines were prepared as follows. Full-length gene fragments encoding human, murine and cynomolgus monkey CCR8 were obtained via full gene synthesis, and each fragment was inserted into the backbone of a stable expression vector to construct expression plasmids. These three plasmids were separately transfected into HEK293 cells, and monoclonal cell lines stably expressing human CCR8, murine CCR8 and cynomolgus monkey CCR8 (HEK293-hCCR8 cells, HEK293-mCCR8 cells and HEK293-cCCR8 cells) were obtained under puromycin selection; these cell lines were used for flow cytometry assays in the present Example.

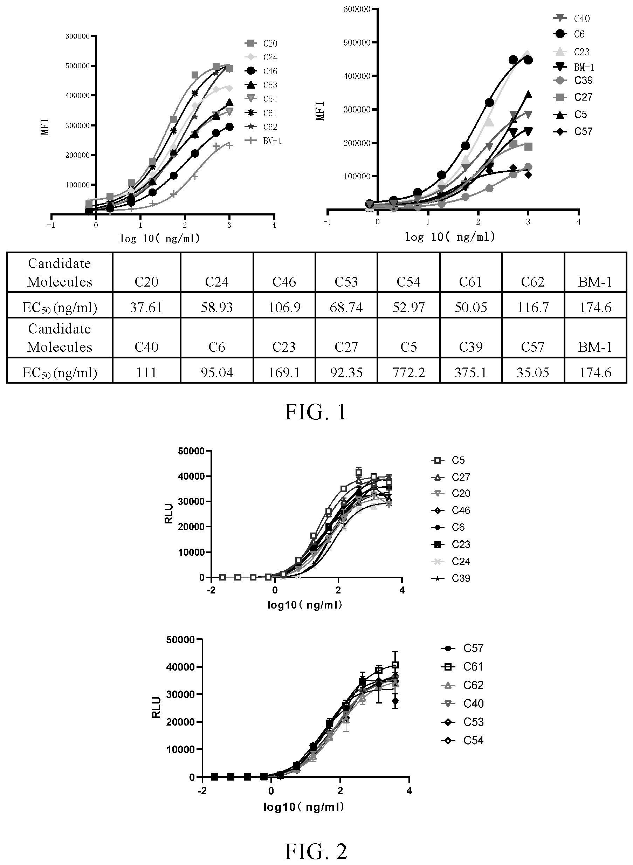

For the binding assay, 1 × 10⁵ HEK293-hCCR8 cells were seeded into each well of a 96-well plate. Antibodies were serially diluted 2-fold starting from an initial concentration of 1 µg/mL. 50 µL of diluted antibody solution was mixed with cells and incubated at 4 °C for 45 min. Cells were washed twice with washing buffer. After discarding the supernatant, cells were resuspended in 50 µL of Goat anti-Mouse IgG PE at a concentration of 200 ng/mL and incubated at 4 °C in the dark for 30 min. Cells were washed three times with washing buffer. Following removal of supernatant, cells were resuspended in 25 µL of dilution buffer and analyzed by flow cytometry. The FACS binding data of the fourteen obtained monoclonal antibodies are shown in FIG. 1.

The results demonstrated that antibodies C20, C24, C46, C53, C54, C61, C62, C40, C6 and C23 exhibited superior Emax and EC50 values compared with BM-1 (antibody sequence disclosed in WO2021194942A1); C57 and C27 showed improved EC50 relative to BM-1; and C5 achieved a higher Emax than BM-1.

Antibody sequences of these positive hybridoma clones were sequenced, cloned into human IgG1 expression vectors, and expressed in FUT8⁻/⁻ CHO cells. Cell culture supernatants were harvested, centrifuged to remove precipitates, filtered through a 0.22 µm filter, and purified using a protein purification system to obtain afucosylated human IgG1 Fc recombinant monoclonal antibodies.

### 1.3 Binding Affinity of Recombinant Human Anti-CCR8 Monoclonal Antibodies to CCR8 Protein

Affinity measurements were performed on the fourteen recombinant human monoclonal antibodies as described below.

Antibodies were diluted to 5 µg/mL. Antigens human CCR8(1-35aa)-mFc and cynomolgus monkey CCR8-mFc were separately diluted to serial concentrations of 50 nM, 25 nM, 12.5 nM, 6.25 nM, 3.12 nM, 1.56 nM, 0.78 nM, or 1000 nM, 500 nM, 250 nM, 125 nM, 62.5 nM, 31.2 nM, 15.6 nM. Wells with zero antigen concentration were included as baselines. Antibody affinity detection was carried out using a Gator instrument.

The results are shown in Table 2. The affinity of candidate antibodies for human CCR8 ranged from 0.0168 nM to 1.24 nM (Table 1). Multiple candidate antibodies were capable of binding to cynomolgus monkey CCR8 (Table 2).

**Table 1. Binding Affinity of Antibodies to Human CCR8**

| Antibody Name | | | KD(M) |
|---|---|---|---|
| | C5 | | 1.78E-10 |
| | C6 | | 1.24E-09 |
| | C20 | | 3.35E-10 |
| | C23 | | 7.05E-10 |
| | C24 | | 1.68E-11 |
| | C27 | | 4.39E-10 |
| | C40 | | 8.08E-10 |
| | C46 | | 1.02E-09 |
| | C53 | | 5.36E-10 |
| | C54 | | 3.80E-10 |
| | C61 | | 4.44E-10 |
| | C62 | | 3.69E-10 |
| | C39 | | NA |
| | C57 | | NA |

**Table 2. Binding of Antibodies to Cynomolgus Monkey CCR8**

| Antibody Name | | +/- |
|---|---|---|
| | BM-1 | + |
| | C5 | - |
| | C6 | + |
| | C20 | + |
| | C23 | + |
| | C24 | + |
| | C27 | - |
| | C40 | + |
| | C46 | + |
| | C53 | + |
| | C54 | + |
| | C61 | + |
| | C62 | + |
| | C39 | - |
| | C57 | - |

### 1.4 ADCC Activity of Recombinant Human Anti-CCR8 Monoclonal Antibodies

The ADCC activity of the fourteen recombinant human anti-CCR8 monoclonal antibodies was determined as follows.

CHO-K1 cells overexpressing CCR8 were seeded at 2 × 10⁴ cells per well and cultured overnight. Culture medium was removed, and 25 µL of complete medium was added to each well. Antibody stock solutions were prepared in complete medium to yield final concentrations of 4000 ng/mL, 1333.3 ng/mL, 444.4 ng/mL, 148.1 ng/mL, 49.38 ng/mL, 16.5 ng/mL, 5.48 ng/mL, 1.82 ng/mL, 0.61 ng/mL, 0.20 ng/mL, 0.06 ng/mL and 0.02 ng/mL. 25 µL of diluted antibody solution was added to each well. The density of Jurkat-FcγRIIIa-V158 effector cells was maintained between 0.2 × 10⁶ cells/mL and 1.0 × 10⁶ cells/mL. Effector cells were resuspended in medium to a density of 6 × 10⁶ cells/mL, and 25 µL of cell suspension was dispensed into each well of a white 96-well plate, resulting in a final volume of 75 µL per sample well. Plates were incubated in a cell incubator for 6 hours. Luciferase substrate was added at 50 µL per well, followed by mixing for 30 seconds. Luminescence signals were acquired using a multi-mode microplate reader via the Lum-TM channel.

The results are shown in FIG. 2. All candidate antibodies exhibited varying degrees of ADCC activity against target cells. Among them, C5 possessed a relatively minimal EC50 value, and C61 generated a relatively maximal signal intensity; both displayed potent ADCC activity.

### 1.5 Inhibitory Effect of Recombinant Human Anti-CCR8 Monoclonal Antibodies on the Binding between CCR8 and Its Ligand CCL1

The inhibitory effect of the fourteen recombinant human anti-CCR8 monoclonal antibodies on the binding of CCR8 to its ligand CCL1 was evaluated using the following method.

Blocking assay was performed using the DiscoverX PathHunter^{®} eXpress β-Arrestin GPCR Assay kit. Briefly, 11.5 mL of Cell Plating Reagent 2 was added into a 15 mL centrifuge tube. 0.5 mL of Cell Plating Reagent was used to thaw the PathHunter^{®} eXpress β-Arrestin GPCR cells; the entire cell suspension was transferred to the above 15 mL centrifuge tube and mixed homogeneously. 100 µL of cell suspension was dispensed into each well, and the plate was incubated in a cell incubator for 48 hours. Antibody solutions were prepared with an initial concentration of 220,000 ng/mL (22-fold of the final concentration), followed by 3-fold serial dilution to generate eight concentration gradients. 5 µL of diluted antibody solution was added to each well of a white plate, and complete medium was added to control wells. The plate was incubated in a cell incubator for 30 minutes. CCL1 solution was prepared at 88 nM (22-fold of the final concentration; the final concentration of CCL1 was 4 nM), and 5 µL was added per well. The plate was incubated in a cell incubator for 90 minutes. Thereafter, 55 µL of Working Detection Solution was added to each well, followed by incubation at room temperature in the dark for 60 minutes. Luminescence signals were detected via the Lum-TM channel.

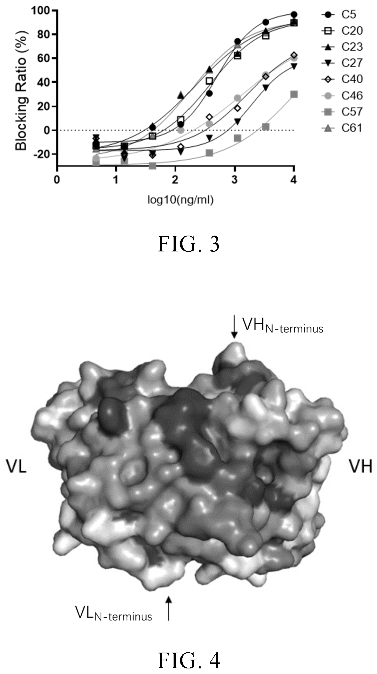

The results are shown in FIG. 3. All tested antibodies exhibited certain inhibitory activity against the binding of CCL1 to CCR8. Antibodies with maximal inhibition exceeding 90% included C5, C20, C23 and C57, with inhibition percentages of 96.83%, 90.08%, 91.39% and 92.46%, respectively; their corresponding IC50 values were 496.8 ng/mL, 377.4 ng/mL, 216.5 ng/mL and 183.4 ng/mL.

### Example 2 Preparation and Characterization of Anti-VEGF Variants

Antibody AS-1 (heavy chain variable region set forth in SEQ ID NO: 89, light chain variable region set forth in SEQ ID NO: 95) exhibits strong intrinsic hydrophobicity and aggregation propensity. To improve the physicochemical properties of the antibody and facilitate subsequent application of this antibody sequence in bispecific or trispecific antibody formats, the inventors designed a series of mutations.

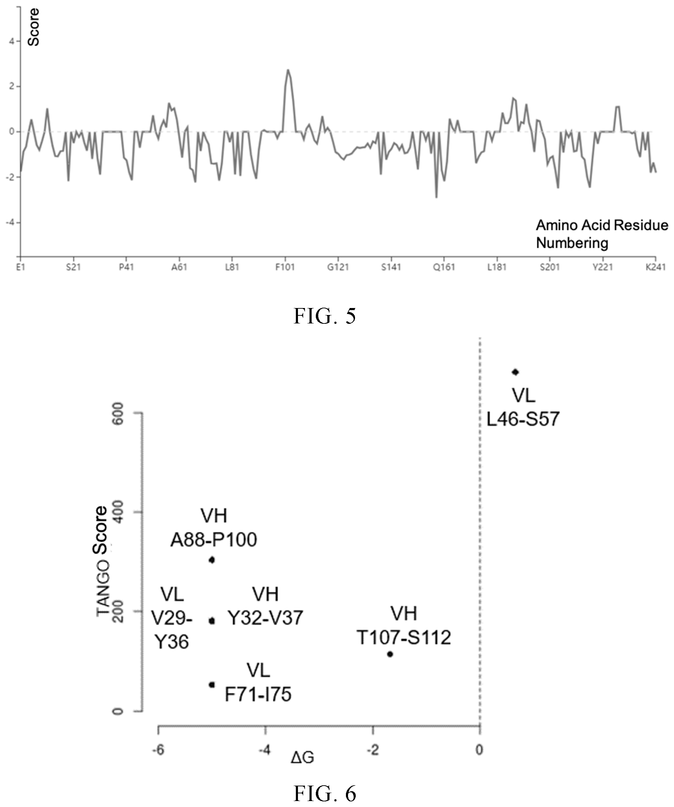

The surface hydrophobic regions of the Fab structure of AS-1 (PDB: 2FJG) were visualized using the color_h.py script available on the PyMOL Wiki. Higher hydrophobicity is indicated by darker gray-black coloring, whereas lower hydrophobicity corresponds to lighter off-white coloring, as shown in FIG. 4. Generally, protein sequences with elevated hydrophobicity tend to aggregate and display reduced protein stability. To further characterize the hydrophobicity of relevant surface amino acids after conversion to the scFv format, the inventors predicted the scFv structure of AS-1 via AlphaFold (the scFv sequence is identical to the AS-1 scFv sequence used in the bispecific and trispecific molecules of the present invention) and analyzed it using the Aggrescan server, as illustrated in FIG. 5. All amino acid residues with scores greater than zero are hydrophobic and confer aggregation propensity to the antibody. Numerous hydrophobic amino acid residues were identified from the Fab and scFv structures of AS-1 and selected as potential mutation sites.

In addition, to simultaneously evaluate aggregation propensity and thermal stability of the AS-1 sequence, the inventors used the SolubiS server to analyze variable region segments of AS-1 that are prone to aggregation (high TANGO scores) and exhibit poor thermal stability (high free energy ΔG). As shown in Figure 6, six peptide segments were identified. Among them, the light chain region L46-S57 demonstrated the highest aggregation propensity and lowest thermal stability. Furthermore, site-directed mutagenesis reported in literature (Dudgeon et al., 2012, PNAS) confirmed that mutations at residues 24, 49, 50, 51, 52, 53 and 56 of the light chain, and residues 28, 30, 31, 32, 33 and 35 of the heavy chain can effectively alleviate antibody aggregation.

Based on the foregoing structural and sequence analyses, the present invention introduced aspartic acid (D) substitutions at selected residues primarily within the L46-S57 region, as well as other regions including structurally identified hydrophobic sites and reported aggregation-prone sites, to reduce hydrophobicity. Moreover, structural information deposited for PDB entry 2FJG reveals that the VH-CDR3 region plays a critical role in VEGF binding, with many residues buried in the protein interior. Accordingly, mutations designed herein are mainly introduced into non-VH-CDR3 regions and other regions irrelevant to VEGF binding to preserve the VEGF-binding activity of the antibody. Seven AS-1 variants were generated via site-directed mutagenesis in the present Example.

To verify whether these mutations alter antibody hydrophobicity, a Proteomix HIC Butyl-NP5 column was employed to differentiate variants according to retention time. Each antibody variant was loaded onto the column in a solution containing 1.5 M NaCl and 25 mM Na₃PO₄ at pH 7.4, followed by elution using a gradient of decreasing salt concentration at pH 7.4. Retention times of the variants are summarized in Table 3. AS-1 exhibited the longest retention time, indicating the strongest hydrophobicity. All other variants displayed reduced retention times. Certain combinatorial mutants such as AS-6 achieved nearly 50% reduction in retention time, substantially lowering antibody hydrophobicity and the associated aggregation risk.

**Table 3**

| Variants | Retention Time (min) |
|---|---|
| AS-1 (ASH, ASL) | 18.78 |
| AS-2 (AS2H, ASL) | 14.91 |
| AS-3 (ASH, AS2L) | 12.90 |
| AS-4 (ASH, AS3L) | 14.68 |
| AS-5(ASH, AS4L) | 11.44 |
| AS-6 (AS2H, AS2L) | 8.69 |
| AS-7 (AS2H, AS3L) | 10.59 |
| AS-8 (AS2H, AS4L) | 7.09 |

To further verify that binding to VEGF was not impaired by the mutations, affinity measurements were performed for each variant. His-tagged human VEGF protein was loaded onto anti-His BLI biosensors. Each variant, prepared in running buffer consisting of 1×PBS, 0.1% BSA, 0.02% Tween-20 and 0.05% sodium azide, was allowed to associate with VEGF for 150 s followed by dissociation for 300 s. The resulting KD values are listed in Table 4. As shown in Table 4, only variants AS-3, AS-6 and AS-8 exhibited markedly reduced binding affinity toward VEGF, whereas the other variants retained favorable affinity comparable to AS-1.

**Table 4**

| Variants | KD(M) |
|---|---|
| AS-1 | 5.15E-11 |
| AS-2 | 3.29E-11 |
| AS-3 | 3.23E-10 |
| AS-4 | 5.14E-11 |
| AS-5 | 8.53E-11 |
| AS-6 | 1.56E-10 |
| AS-7 | 5.37E-11 |
| AS-8 | 4.61E-10 |

### Example 3 Preparation of Multispecific Antibody Molecules

A variety of multispecific antibody molecules were designed based on the fourteen distinct anti-CCR8 antibody sequences obtained in Example 1.

Gene fragments encoding the multispecific antibody sequences were obtained by full gene synthesis, and the target sequences were inserted into expression vectors using standard gene cloning techniques (see, e.g., Lo, B.K.C., Methods in Molecular Biology, Volume 248, 2004, Antibody Engineering).

HEK293E cells were transfected with vectors encoding the multispecific antibody molecules. Cells were cultured in F17 medium (1 L F17 + 10 mL 10% PF68 + 30 mL 200 mM L-glutamine) at 37 °C with 5% CO₂ for 7 days to produce the target molecules. After expression, culture supernatants were harvested and purified to obtain multispecific antibody molecules for subsequent experimental analysis.

Anti-VEGF sequences were derived from molecule AVA (heavy chain variable region set forth in SEQ ID NO: 87, light chain variable region set forth in SEQ ID NO: 93) and variants of the VEGF antibody variable regions and their CDRs disclosed in US7758859. Anti-PD-L1 sequences were derived from molecule PL1 (heavy chain variable region set forth in SEQ ID NO: 103, light chain variable region set forth in SEQ ID NO: 107) or PL2 (heavy chain variable region set forth in SEQ ID NO: 105, light chain variable region set forth in SEQ ID NO: 109).

Multispecific molecules were prepared using the above-described methods as follows:
(1) Bispecific antibodies composed of a CCR8 antigen-binding domain and a VEGF or PD-L1 antigen-binding domain with formats illustrated in a-i of FIG. 7A.
   An exemplary bispecific antibody adopting format a in FIG. 7A is designated 8As-1, and its structure is shown in FIG. 7B;
   an exemplary bispecific antibody adopting format b in FIG. 7A is designated 8As-2, and its structure is shown in FIG. 7C;
   an exemplary bispecific antibody adopting format c in FIG. 7A is designated 8As-3, and its structure is shown in FIG. 7D;
   an exemplary bispecific antibody adopting format d in FIG. 7A is designated 8As-4, and its structure is shown in FIG. 7E;
   an exemplary bispecific antibody adopting format e in FIG. 7A is designated 8As-5, and its structure is shown in FIG. 7F;
   an exemplary bispecific antibody adopting format f in FIG. 7A is designated 8As-6, and its structure is shown in FIG. 7G;
   an exemplary bispecific antibody adopting format g in FIG. 7A is designated 8As-7, and its structure is shown in FIG. 7H;
   an exemplary bispecific antibody adopting format h in FIG. 7A is designated Pl8-8, and its structure is shown in FIG. 7I;
   one exemplary bispecific antibody adopting format i in FIG. 7A is designated Pl8-9, and its structure is shown in FIG. 7J; another bispecific antibody adopting format i is designated 8As-9, and its structure is shown in FIG. 7K. The difference from Pl8-9 lies in replacement of the anti-PD-L1 VH and VL with anti-VEGF VH and VL.
(2) Trispecific antibodies composed of a CCR8 antigen-binding domain, a VEGF antigen-binding domain and a PD-L1 antigen-binding domain with formats illustrated in a-d of FIG. 8A.
   An exemplary trispecific antibody adopting format a in FIG. 8A is designated 8AsPl-1, and its structure is shown in FIG. 8B;
   an exemplary trispecific antibody adopting format b in FIG. 8A is designated 8AsPl-2, and its structure is shown in FIG. 8C;
   an exemplary trispecific antibody adopting format c in FIG. 8A is designated 8AsPl-3, and its structure is shown in FIG. 8D;
   an exemplary trispecific antibody adopting format d in FIG. 8A is designated 8AsPl-4 or 8AsPl-4v, and its structure is shown in FIG. 8E.

Exemplary molecules prepared in this Example are listed below, wherein the VH and VL of C61 and/or C27 are employed for the CCR8 antigen-binding domain.

| Molecule Name | Chain 1 (SEQ ID NO:) | | Chain 2 (SEQ ID NO:) | | Chain 3 (SEQ ID NO:) | | Chain 4 (SEQ ID NO:) | |
|---|---|---|---|---|---|---|---|---|
| 8As-1 | | 124 | | 125 | | 126 | | 127 |
| 8As-2 | | 124 | | 128 | | / | | / |
| 8As-3 | | 124 | | 128 | | 125 | | / |
| 8As-4 | | 124 | | 129 | | 125 | | / |
| 8As-5 | | 124 | | 130 | | 125 | | / |
| 8As-6 | | 131 | | 132 | | 133 | | 134 |
| 8As-7 | | 131 | | 132 | | 135 | | 134 |
| Pl8-8 | | 124 | | 136 | | / | | / |
| Pl8-9 | | 137 | | 138 | | 139 | | / |
| 8As-9 | | 137 | | 140 | | 134 | | / |
| 8AsPl-1 | | 124 | | 141 | | 142 | | / |
| 8AsPl-2 | | 143 | | 144 | | 145 | | 134 |
| 8AsPl-3 | | 124 | | 142 | | 146 | | / |
| 8AsPl-4 | | 124 | | 147 | | / | | / |
| 8AsPl-4v | | 124 | | 148 | | | | |

The structure of molecule 8AsPl-4v is identical to that of 8AsPl-4. The distinction lies in that the anti-VEGF antigen-binding domain thereof comprises the VH set forth in SEQ ID NO: 91 and the VL set forth in SEQ ID NO: 99, and the C-terminal lysine (K) of the Fc region is truncated.

### Example 4 Functional Characterization of Multispecific Antibodies Targeting CCR8 and VEGF/PD-L1

### 4.1 ADCC Assay for Multispecific Antibodies Targeting CCR8 and VEGF/PD-L1

ADCC effects of the multispecific antibodies targeting CCR8 and VEGF/PD-L1 prepared in Example 3 (8As-1, 8As-2, 8As-4, 8As-7, 8As-9, Pl8-8, Pl8-9, 8AsPl-1, 8AsPl-3, 8AsPl-4) were evaluated. Some antibodies were expressed in FUT8-knockout CHO cells (FKO). The detailed procedure is described below.

CHOK1-hCCR8 cells in logarithmic growth phase (human CCR8-overexpressing cells generated by transfecting CHOK1 cells with full-length CCR8 expression plasmid, prepared following the same protocol used for HEK293-hCCR8 cells described in Example 1.2) were harvested and centrifuged at 300 × g for 5 min. Cells were resuspended in 1 mL assay buffer (RPMI 1640 medium supplemented with 10% FBS), counted, and adjusted to a density of 1 × 10⁶ cells/mL using assay buffer. After thorough mixing, 25 µL of CHOK1-hCCR8 cell suspension was dispensed into each well of a white 96-well plate. Serially diluted antibodies prepared in assay buffer were added to the plate at 25 µL per well, with technical duplicates set up. The plate was incubated in a CO₂ incubator for 30 min. ADCC Bioassay Effector Cell V Variant (High Affinity)/NFAT Luciferase Reporter Jurkat Cell Line with good viability was collected into a 50 mL centrifuge tube and centrifuged at 300 × g for 5 min. Cells were resuspended in 3 mL assay buffer (RPMI 1640 medium + 10% FBS), counted and adjusted to a density of 3 × 10⁶ cells/mL. After mixing, 50 µL of effector cell suspension was added into each well of the above 96-well plate. The plate was incubated in a CO₂ incubator for 5 h. BRITELITE PLUS reagent was added at 100 µL per well. Following incubation at room temperature in the dark for 5 min, luminescence signals were measured using a microplate reader. Data were plotted and EC50 values were calculated using GraphPad Prism software.

The results presented in FIG. 9 demonstrate that antibodies expressed in FUT8-knockout CHO cells (labeled FKO after the molecule name) exhibited markedly enhanced ADCC activity compared with the identical antibodies expressed in wild-type CHO cells (FIG. 9, panel e). Bispecific antibodies with multiple formats displayed potent ADCC activity (FIG. 9, panels a-d). Among them, 8As-1, 8As-4, Pl8-8 and Pl8-9 exerted ADCC activity against CCR8 comparable to the parental monoclonal antibody; their bispecific architecture did not impair ADCC function. The trispecific molecule 8AsPl-4 exhibited favorable ADCC activity against CCR8, while its ADCC activity toward PD-L1 was very weak (FIG. 9, panels f and g).

### 4.2 VEGF Inhibition Assay of Multispecific Antibodies Targeting CCR8 and VEGF/PD-L1

The capacity of multispecific antibodies targeting CCR8 and VEGF/PD-L1 (8As-1, 8As-2, 8As-4, 8As-9, 8AsPl-1, 8AsPl-3, 8AsPl-4) to block VEGF signaling was evaluated, with molecule AS-1 set as the control sample. The detailed procedure is described below.

VEGFR2/NFAT Reporter-HEK293 Recombinant cells in logarithmic growth phase were harvested and centrifuged at 300 × g for 5 min. Cells were resuspended in 1 mL assay buffer (MEM/EBSS medium supplemented with 10% FBS), counted, and adjusted to a density of 6 × 10⁵ cells/mL using assay buffer. After thorough mixing, 50 µL of the cell suspension was dispensed into each well of a white 96-well plate. Serially diluted antibodies prepared in assay buffer were added to the plate at 25 µL per well with technical duplicates. Human His-tagged VEGF₁₆₅ protein was diluted to 50 ng/mL in assay buffer, and 25 µL was transferred into each well. The plate was incubated in a CO₂ incubator for 4 h. Subsequently, BRITELITE PLUS reagent was added at 100 µL per well. After incubation at room temperature in the dark for 5 min, luminescence signals were measured using a microplate reader. Data were plotted and IC₅₀ values were calculated using GraphPad Prism software.

The results are shown in FIG. 10. FIG. 10, panels a and b illustrate that all tested bispecific antibodies exhibited comparable VEGF inhibitory activity with marginal differences. Among trispecific molecules (FIG. 10, panel c), 8AsPl-4 displayed potent inhibitory activity similar to the parental monoclonal antibody.

### 4.3 PD-L1 Blocking Assay of Multispecific Antibodies Targeting CCR8 and VEGF/PD-L1

The ability of multispecific antibodies targeting CCR8 and VEGF/PD-L1 to block the binding between PD-1 and PD-L1 was determined, with molecule PL1 used as the control sample. The detailed procedure is described below.

CHO-hPD-L1 cells in logarithmic growth phase were collected and centrifuged at 300 × g for 5 min. Cells were resuspended in 1 mL FACS buffer (1 × PBS + 2% FBS), counted, and adjusted to a density of 2 × 10⁶ cells/mL using FACS buffer. After mixing evenly, 25 µL of CHO-hPD-L1 cell suspension was added to each well of a 96-well V-bottom plate. Serially diluted antibodies (8AsPl-1, 8AsPl-3, 8AsPl-4, or Pl8-8-FKO, Pl8-9-FKO) were dispensed at 50 µL per well with technical duplicates. Human PD-1-mFc fusion protein was diluted to 16 µg/mL in FACS buffer, and 25 µL was added to each well. The plate was incubated at 4 °C for 30 min. Cells were washed twice with FACS buffer, followed by addition of Goat anti-Mouse IgG Fc Cross-Adsorbed PE-conjugated secondary antibody, and incubation continued at 4 °C for 20 min. After two further washes with FACS buffer, samples were analyzed by flow cytometry. Experimental data were plotted and IC₅₀ values were calculated using GraphPad Prism software.

The results are presented in FIG. 11. The two bispecific antibodies achieved PD-L1 blocking activity substantially equivalent to the control monoclonal antibody (FIG. 11, panel a). Among trispecific molecules, 8AsPl-3 and 8AsPl-4 exhibited stronger blocking activity than 8AsPl-1, yet weaker than the monoclonal antibody control (FIG. 11, panel b).

### Example 5 In Vivo Efficacy Studies of Antibody Molecules Targeting CCR8

### 5.1 In Vivo Efficacy in the MC38 Mouse Model

CCR8 humanized C57BL/6 mice were inoculated with MC38 cells (5 × 10⁵ cells per mouse) to establish the MC38 tumor model. Once the mean tumor volume reached approximately 100 mm³, mice were randomized into groups and treatment was initiated. Antibodies were administered intravenously at a dose of 10 mg/kg twice weekly for 5 consecutive doses.

The results are shown in FIG. 12, panel a. On Day 14 after the first administration, BM-1, C61 and C27 exhibited anti-tumor activity, with tumor growth inhibition (TGI) values of 30%, 34% and 45%, respectively.

In a separate experiment, CCR8 humanized C57BL/6 mice were inoculated with MC38 cells using the same cell inoculum, tumor randomization volume and equimolar dosing as the experiment shown in FIG. 12, panel a. Treatments were administered intraperitoneally twice weekly for 6 consecutive doses.

As presented in FIG. 12, panel b, on Day 20 post-administration, C61, 8As-2 and 8AsPl-4v demonstrated anti-tumor efficacy, with TGI values of 45%, 83.5% and 89.5%, respectively.

### 5.2 In Vivo Efficacy in the CT26 Mouse Model

CCR8 humanized Balb/c mice were inoculated with CT26 cells (3 × 10⁵ cells per mouse) to establish the CT26 tumor model. Grouping and treatment were initiated when the mean tumor volume reached approximately 60 mm³. Antibodies were administered intraperitoneally at doses outlined in FIG. 13 twice weekly for 4 consecutive doses.

The results are shown in FIG. 13, panel a. On Day 14 after treatment initiation, the tumor growth inhibition (TGI) values for each treatment group were 70% (AS-1), 4% (C61), 66% (AS-1 + C61), 63% (8As-1), 58% (8As-2) and 74% (8As-4). Body weight changes of mice throughout the treatment period are illustrated in FIG. 13, panel b. Collectively, the bispecific molecules of the present invention displayed potent anti-tumor efficacy *in vivo*.

All references mentioned in the present application are incorporated by reference herein, as though individually incorporated by reference. In addition, it should be understood that after reading the above teaching content of the present invention, various changes or modifications may be made by those skilled in the art, and these equivalents also fall within the scope as defined by the appended claims of the present application.

## Claims

1. An anti-CCR8 antibody or antigen-binding fragment thereof, wherein the antibody comprises the following three heavy chain variable region CDRs:
HCDR1 having an amino acid sequence set forth in SEQ ID NO: 1, 4, 7, 10, 15, 18, 20, 24, 28 or 30;
HCDR2 having an amino acid sequence set forth in SEQ ID NO: 2, 5, 8, 11, 13, 16, 21, 23, 25 or 31; and
HCDR3 having an amino acid sequence set forth in SEQ ID NO: 3, 6, 9, 12, 14, 17, 19, 22, 26, 27, 29 or 32;
and the following three light chain variable region CDRs:
LCDR1 having an amino acid sequence set forth in SEQ ID NO: 33, 36, 39, 50 or 55;
LCDR2 having an amino acid sequence set forth in SEQ ID NO: 34, 37, 40, 44, 48, 51, 53, 56 or 58; and
LCDR3 having an amino acid sequence set forth in SEQ ID NO: 35, 38, 42, 45, 49, 52, 54 or 57.

2. The anti-CCR8 antibody or antigen-binding fragment thereof according to claim 1, wherein the anti-CCR8 antibody or antigen-binding fragment thereof comprises a heavy chain variable region having at least 80% sequence identity to the amino acid sequence set forth in any one of SEQ ID NOs: 59-72, and/or the light chain variable region having at least 80% sequence identity to an amino acid sequence set forth in any one of SEQ ID NOs: 73-86.

3. A multispecific antibody, wherein the multispecific antibody comprises:
a first targeting domain, wherein the first targeting domain comprises one or more CCR8 antigen-binding domains, and the CCR8 antigen-binding domain comprises the anti-CCR8 antibody or antigen-binding fragment thereof according to claim 1;
a second targeting domain, wherein the second targeting domain binds to VEGF or PD-L1;
optionally, a third targeting domain, wherein the third targeting domain binds to VEGF or PD-L1;
and the second targeting domain and the third targeting domain bind to different antigens respectively.

4. The multispecific antibody according to claim 3, wherein the targeting domain is in the form of a single-chain Fv (scFv), Fab fragment, single-domain antibody (sdAb), variable fragment (Fv) heterodimer, TriFab, or a combination thereof.

5. The multispecific antibody according to claim 3, wherein the VEGF antigen-binding domain comprises a heavy chain variable region having at least 80% sequence identity to the amino acid sequence set forth in SEQ ID NO: 87 or 88, and a light chain variable region having at least 80% sequence identity to the amino acid sequence set forth in SEQ ID NO: 93 or 94; or
comprises a heavy chain variable region having at least 80% sequence identity to the amino acid sequence set forth in any one of SEQ ID NO: 89-92 and 149-150, and a light chain variable region having at least 80% sequence identity to the amino acid sequence set forth in any one of SEQ ID NO: 95-102.

6. The multispecific antibody according to claim 3, wherein the PD-L1 antigen-binding domain comprises a heavy chain variable region having at least 80% sequence identity to the amino acid sequence set forth in SEQ ID NO: 103 or 104, and a light chain variable region having at least 80% sequence identity to the amino acid sequence set forth in SEQ ID NO: 107 or 108; or
comprises a heavy chain variable region having at least 80% sequence identity to the amino acid sequence set forth in SEQ ID NO: 105 or 106, and a light chain variable region having at least 80% sequence identity to the amino acid sequence set forth in SEQ ID NO: 109 or 110.

7. The multispecific antibody according to claim 3, wherein the multispecific antibody further comprises an Fc fragment; preferably, the Fc fragment is derived from IgG1 or IgG4.

8. The multispecific antibody according to claim 7, wherein the Fc fragment comprises mutations for forming a knob-in-hole structure and/or mutations for enhancing ADCC.

9. The multispecific antibody according to claim 3, wherein the multispecific antibody is a bispecific or trispecific antibody.

10. A polynucleotide encoding the anti-CCR8 antibody or antigen-binding fragment thereof according to claim 1, or the multispecific antibody according to claim 3.

11. Use of the anti-CCR8 antibody or antigen-binding fragment thereof according to claim 1, or the multispecific antibody according to claim 3, for the manufacture of a medicament for treating cancer/tumor.

12. An immunoconjugate, wherein the immunoconjugate comprises:
(i) the anti-CCR8 antibody or antigen-binding fragment thereof according to claim 1, or the multispecific antibody according to claim 3; and
(ii) a conjugate moiety selected from the group consisting of a detectable label, a drug, a toxin, a cytokine, a radionuclide and an enzyme.

13. A pharmaceutical composition, wherein the pharmaceutical composition comprises: (a) the anti-CCR8 antibody or antigen-binding fragment thereof according to claim 1, or the multispecific antibody according to claim 3; and (b) a pharmaceutically acceptable carrier.

14. Use of the anti-CCR8 antibody or antigen-binding fragment thereof according to claim 1, or the immunoconjugate according to claim 12, for the manufacture of a detection reagent or kit, wherein the detection reagent or kit is used for detecting CCR8 molecules in a sample.

15. An anti-VEGF antibody variant, wherein the anti-VEGF antibody variant comprises a heavy chain variable region having at least 80% sequence identity to the amino acid sequence set forth in SEQ ID NO: 89 and a light chain variable region having at least 80% sequence identity to the amino acid sequence set forth in SEQ ID NO: 95, and comprises mutations capable of reducing antibody hydrophobicity.
